# EUROPEAN PATENT SPECIFICATION

(11) **EP 1 467 207 B1**
(45) Date of publication and mention of the grant of the patent: **04.06.2008**
(21) Application number: 03701036.0
(22) Date of filing: 09.01.2003
(51) Int. Cl.: G01N 33/68, G01N 33/50

(54) **Screening method for a prophylactic and therapeutic substance for a renal disease.**
Screening-verfahren für eine prophylaktische und therapeutische Substanz für eine Nierenerkrankung
Méthode de criblage pour un composé prophylactique et thérapeutique pour une maladie rénale.

(30) Priority: 10.01.2002 JP 2002003769
(43) Date of publication of application: 13.10.2004
(73) Proprietor: Takeda Pharmaceutical Company Limited, Osaka (JP)
(72) Inventor: NODA, Masakuni, Takarazuka-shi, Hyogo 665-0861 (JP); MATSUO, Takanori, Ikeda-shi, Osaka 563-0026 (JP); TSUGE, Hiroko, Nishinomiya-shi, Hyogo 663-8106 (JP)
(74) Representative: Rickard, Timothy Mark Adrian
(86) International application number: PCT/JP2003/000111
(87) International publication number: WO 2003/060516

(56) References cited:
- WO-A-01/04356
- WO-A-01/30394
- WO-A-89/09777
- WO-A-94/23030
- WO-A1-01/04356
- WO-A2-01/74298
- WO-A2-99/62561
- JP-A- 2000 506 725
- US-A- 6 008 048
- RUPPRECHT H D ET AL: "Expression of the transcriptional regulator Egr-1 in experimental glomerulonephritis: requirement for mesangial cell proliferation" KIDNEY INTERNATIONAL, NEW YORK, NY, US, vol. 51, no. 3, 1997, pages 694-702, XP008029558 ISSN: 0085-2538
- SWIRNOFF ALEXANDER H ET AL: "DNA-binding specificity of NGFI-A and related zinc finger transcription factors" MOLECULAR AND CELLULAR BIOLOGY, vol. 15, no. 4, 1995, pages 2275-2287, XP002376477 ISSN: 0270-7306
- POPOU B., KACZMARER L. BIOCHEMISTRY AND MOLECULAR BIOLOGY INTERNATIONAL vol. 32, no. 1, 1994, pages 39 - 47, XP002967748

### SEQUENCE LISTING

<110> Takeda Chemical Industries, Ltd.
<120> Screening Method
<130> P04-080PCT
<150> JP 2002-3769
   <151> 2002-01-10
<160> 11
<170> PatentIn version 3.1
<210> 1
   <211> 123
   <212> PRT
   <213> Homo sapiens
<220>
   <221> MISC_FEATURE
   <223> Partial amino acid sequence of Egr-1 protein which is conserved between human, mouse and rat.
<400> 1
   Tyr Gln Ser Gln Leu Ile Lys Pro Ser Arg Met Arg Lys Tyr Pro Asn
   1               5                   10                  15
   Arg Pro Ser Lys Thr Pro Pro His Glu Arg Pro Tyr Ala Cys Pro Val
               20                  25                  30
   Glu Ser Cys Asp Arg Arg Phe Ser Arg Ser Asp Glu Leu Thr Arg His
           35                  40                  45
   Ile Arg Ile His Thr Gly Gln Lys Pro Phe Gln Cys Arg Ile Cys Met
       50                  55                  60
   Arg Asn Phe Ser Arg Ser Asp His Leu Thr Thr His Ile Arg Thr His
   65                  70                  75                  80
   Thr Gly Glu Lys Pro Phe Ala Cys Asp Ile Cys Gly Arg Lys Phe Ala
                   85                  90                  95
   Arg Ser Asp Glu Arg Lys Arg His Thr Lys Ile His Leu Arg Gln Lys
               100                 105                 110
   Asp Lys Lys Ala Asp Lys Ser Val Val Ala Ser
           115                 120
<210> 2
   <211> 543
   <212> PRT
   <213> Homo sapiens
<400> 2
   Met Ala Ala Ala Lys Ala Glu Met Gln Leu Met Ser Pro Leu Gln Ile
   1               5                   10                  15
   Ser Asp Pro Phe Gly Ser Phe Pro His Ser Pro Thr Met Asp Asn Tyr
               20                  25                  30
   Pro Lys Leu Glu Glu Met Met Leu Leu Ser Asn Gly Ala Pro Gln Phe
           35                  40                  45
   Leu Gly Ala Ala Gly Ala Pro Glu Gly Ser Gly Ser Asn Ser Ser Ser
       50                  55                  60
   Ser Ser Ser Gly Gly Gly Gly Gly Gly Gly Gly Gly Ser Asn Ser Ser
   65                  70                  75                  80
   Ser Ser Ser Ser Thr Phe Asn Pro Gln Ala Asp Thr Gly Glu Gln Pro
                   85                  90                  95
   Tyr Glu His Leu Thr Ala Glu Ser Phe Pro Asp Ile Ser Leu Asn Asn
               100                 105                 110
   Glu Lys Val Leu Val Glu Thr Ser Tyr Pro Ser Gln Thr Thr Arg Leu
           115                 120                 125
   Pro Pro Ile Thr Tyr Thr Gly Arg Phe Ser Leu Glu Pro Ala Pro Asn
       130                 135                 140
   Ser Gly Asn Thr Leu Trp Pro Glu Pro Leu Phe Ser Leu Val Ser Gly
   145                 150                 155                 160
   Leu Val Ser Met Thr Asn Pro Pro Ala Ser Ser Ser Ser Ala Pro Ser
                   165                 170                 175
   Pro Ala Ala Ser Ser Ala Ser Ala Ser Gln Ser Pro Pro Leu Ser Cys
               180                 185                 190
   Ala Val Pro Ser Asn Asp Ser Ser Pro Ile Tyr Ser Ala Ala Pro Thr
           195                 200                 205
   Phe Pro Thr Pro Asn Thr Asp Ile Phe Pro Glu Pro Gln Ser Gln Ala
       210                 215                 220
   Phe Pro Gly Ser Ala Gly Thr Ala Leu Gln Tyr Pro Pro Pro Ala Tyr
   225                 230                 235                 240
   Pro Ala Ala Lys Gly Gly Phe Gln Val Pro Met Ile Pro Asp Tyr Leu
                   245                 250                 255
   Phe Pro Gln Gln Gln Gly Asp Leu Gly Leu Gly Thr Pro Asp Gln Lys
               260                 265                 270
   Pro Phe Gln Gly Leu Glu Ser Arg Thr Gln Gln Pro Ser Leu Thr Pro
           275                 280                 285
   Leu Ser Thr Ile Lys Ala Phe Ala Thr Gln Ser Gly Ser Gln Asp Leu
       290                 295                 300
   Lys Ala Leu Asn Thr Ser Tyr Gln Ser Gln Leu Ile Lys Pro Ser Arg
   305                 310                 315                 320
   Met Arg Lys Tyr Pro Asn Arg Pro Ser Lys Thr Pro Pro His Glu Arg
                   325                 330                 335
   Pro Tyr Ala Cys Pro Val Glu Ser Cys Asp Arg Arg Phe Ser Arg Ser
               340                 345                 350
   Asp Glu Leu Thr Arg His Ile Arg Ile His Thr Gly Gln Lys Pro Phe
           355                 360                 365
   Gln Cys Arg Ile Cys Met Arg Asn Phe Ser Arg Ser Asp His Leu Thr
       370                 375                 380
   Thr His Ile Arg Thr His Thr Gly Glu Lys Pro Phe Ala Cys Asp Ile
   385                 390                 395                 400
   Cys Gly Arg Lys Phe Ala Arg Ser Asp Glu Arg Lys Arg His Thr Lys
                   405                 410                 415
   Ile His Leu Arg Gln Lys Asp Lys Lys Ala Asp Lys Ser Val Val Ala
               420                 425                 430
   Ser Ser Ala Thr Ser Ser Leu Ser Ser Tyr Pro Ser Pro Val Ala Thr
           435                 440                 445
   Ser Tyr Pro Ser Pro Val Thr Thr Ser Tyr Pro Ser Pro Ala Thr Thr
       450                 455                 460
   Ser Tyr Pro Ser Pro Val Pro Thr Ser Phe Ser Ser Pro Gly Ser Ser
   465                 470                 475                 480
   Thr Tyr Pro Ser Pro Val His Ser Gly Phe Pro Ser Pro Ser Val Ala
                   485                 490                 495
   Thr Thr Tyr Ser Ser Val Pro Pro Ala Phe Pro Ala Gln Val Ser Ser
               500                 505                 510
   Phe Pro Ser Ser Ala Val Thr Asn Ser Phe Ser Ala Ser Thr Gly Leu
           515                 520                 525
   Ser Asp Met Thr Ala Thr Phe Ser Pro Arg Thr Ile Glu Ile Cys
       530                 535                 540
<210> 3
   <211> 1629
   <212> DNA
   <213> Homo sapiens
<220>
   <221> CDS
   <222> (1)..(1629)
   <223>
<400> 3
   atg gcc gcg gcc aag gcc gag atg cag ctg atg tcc ccg ctg cag atc      48
   Met Ala Ala Ala Lys Ala Glu Met Gln Leu Met Ser Pro Leu Gln Ile
   1               5                   10                  15
   tct gac ccg ttc gga tcc ttt cct cac tcg ccc acc atg gac aac tac      96
   Ser Asp Pro Phe Gly Ser Phe Pro His Ser Pro Thr Met Asp Asn Tyr
               20                  25                  30
   cct aag ctg gag gag atg atg ctg ctg agc aac ggg gct ccc cag ttc     144
   Pro Lys Leu Glu Glu Met Met Leu Leu Ser Asn Gly Ala Pro Gln Phe
           35                  40                  45
   ctc ggc gcc gcc ggg gcc cca gag ggc agc ggc agc aac agc agc agc     192
   Leu Gly Ala Ala Gly Ala Pro Glu Gly Ser Gly Ser Asn Ser Ser Ser
       50                  55                  60
   agc agc agc ggg ggc ggt gga ggc ggc ggg ggc ggc agc aac agc agc     240
   Ser Ser Ser Gly Gly Gly Gly Gly Gly Gly Gly Gly Ser Asn Ser Ser
   65                  70                  75                  80
   agc agc agc agc acc ttc aac cct cag gcg gac acg ggc gag cag ccc     288
   Ser Ser Ser Ser Thr Phe Asn Pro Gln Ala Asp Thr Gly Glu Gln Pro
                   85                  90                  95
   tac gag cac ctg acc gca gag tct ttt cct gac atc tct ctg aac aac     336
   Tyr Glu His Leu Thr Ala Glu Ser Phe Pro Asp Ile Ser Leu Asn Asn
               100                 105                 110
   gag aag gtg ctg gtg gag acc agt tac ccc agc caa acc act cga ctg     384
   Glu Lys Val Leu Val Glu Thr Ser Tyr Pro Ser Gln Thr Thr Arg Leu
           115                 120                 125
   ccc ccc atc acc tat act ggc cgc ttt tcc ctg gag cct gca ccc aac     432
   Pro Pro Ile Thr Tyr Thr Gly Arg Phe Ser Leu Glu Pro Ala Pro Asn
       130                 135                 140
   agt ggc aac acc ttg tgg ccc gag ccc ctc ttc agc ttg gtc agt ggc     480
   Ser Gly Asn Thr Leu Trp Pro Glu Pro Leu Phe Ser Leu Val Ser Gly
   145                 150                 155                 160
   cta gtg agc atg acc aac cca ccg gcc tcc tcg tcc tca gca cca tct     528
   Leu Val Ser Met Thr Asn Pro Pro Ala Ser Ser Ser Ser Ala Pro Ser
                   165                 170                 175
   cca gcg gcc tcc tcc gcc tcc gcc tcc cag agc cca ccc ctg agc tgc     576
   Pro Ala Ala Ser Ser Ala Ser Ala Ser Gln Ser Pro Pro Leu Ser Cys
               180                 185                 190
   gca gtg cca tcc aac gac agc agt ccc att tac tca gcg gca ccc acc     624
   Ala Val Pro Ser Asn Asp Ser Ser Pro Ile Tyr Ser Ala Ala Pro Thr
           195                 200                 205
   ttc ccc acg ccg aac act gac att ttc cct gag cca caa agc cag gcc     672
   Phe Pro Thr Pro Asn Thr Asp Ile Phe Pro Glu Pro Gln Ser Gln Ala
       210                 215                 220
   ttc ccg ggc tcg gca ggg aca gcg ctc cag tac ccg cct cct gcc tac     720
   Phe Pro Gly Ser Ala Gly Thr Ala Leu Gln Tyr Pro Pro Pro Ala Tyr
   225                 230                 235                 240
   cct gcc gcc aag ggt ggc ttc cag gtt ccc atg atc ccc gac tac ctg     768
   Pro Ala Ala Lys Gly Gly Phe Gln Val Pro Met Ile Pro Asp Tyr Leu
                   245                 250                 255
   ttt cca cag cag cag ggg gat ctg ggc ctg ggc acc cca gac cag aag     816
   Phe Pro Gln Gln Gln Gly Asp Leu Gly Leu Gly Thr Pro Asp Gln Lys
               260                 265                 270
   ccc ttc cag ggc ctg gag agc cgc acc cag cag cct tcg cta acc cct     864
   Pro Phe Gln Gly Leu Glu Ser Arg Thr Gln Gln Pro Ser Leu Thr Pro
           275                 280                 285
   ctg tct act att aag gcc ttt gcc act cag tcg ggc tcc cag gac ctg     912
   Leu Ser Thr Ile Lys Ala Phe Ala Thr Gln Ser Gly Ser Gln Asp Leu
       290                 295                 300
   aag gcc ctc aat acc agc tac cag tcc cag ctc atc aaa ccc agc cgc     960
   Lys Ala Leu Asn Thr Ser Tyr Gln Ser Gln Leu Ile Lys Pro Ser Arg
   305                 310                 315                 320
   atg cgc aag tat ccc aac cgg ccc agc aag acg ccc ccc cac gaa cgc    1008
   Met Arg Lys Tyr Pro Asn Arg Pro Ser Lys Thr Pro Pro His Glu Arg
                   325                 330                 335
   cct tac gct tgc cca gtg gag tcc tgt gat cgc cgc ttc tcc cgc tcc      1056
   Pro Tyr Ala Cys Pro Val Glu Ser Cys Asp Arg Arg Phe Ser Arg Ser
               340                 345                 350
   gac gag ctc acc cgc cac atc cgc atc cac aca ggc cag aag ccc ttc      1104
   Asp Glu Leu Thr Arg His Ile Arg Ile His Thr Gly Gln Lys Pro Phe
           355                 360                 365
   cag tgc cgc atc tgc atg cgc aac ttc agc cgc agc gac cac ctc acc      1152
   Gln Cys Arg Ile Cys Met Arg Asn Phe Ser Arg Ser Asp His Leu Thr
       370                 375                 380
   acc cac atc cgc acc cac aca ggc gaa aag ccc ttc gcc tgc gac atc      1200
   Thr His Ile Arg Thr His Thr Gly Glu Lys Pro Phe Ala Cys Asp Ile
   385                 390                 395                 400
   tgt gga aga aag ttt gcc agg agc gat gaa cgc aag agg cat acc aag      1248
   Cys Gly Arg Lys Phe Ala Arg Ser Asp Glu Arg Lys Arg His Thr Lys
                   405                 410                 415
   atc cac ttg cgg cag aag gac aag aaa gca gac aaa agt gtt gtg gcc      1296
   Ile His Leu Arg Gln Lys Asp Lys Lys Ala Asp Lys Ser Val Val Ala
               420                 425                 430
   tct tcg gcc acc tcc tct ctc tct tcc tac ccg tcc ccg gtt gct acc      1344
   Ser Ser Ala Thr Ser Ser Leu Ser Ser Tyr Pro Ser Pro Val Ala Thr
           435                 440                 445
   tct tac ccg tcc ccg gtt act acc tct tat cca tcc ccg gcc acc acc      1392
   Ser Tyr Pro Ser Pro Val Thr Thr Ser Tyr Pro Ser Pro Ala Thr Thr
       450                 455                 460
   tca tac cca tcc cct gtg ccc acc tcc ttc tcc tct ccc ggc tcc tcg      1440
   Ser Tyr Pro Ser Pro Val Pro Thr Ser Phe Ser Ser Pro Gly Ser Ser
   465                 470                 475                 480
   acc tac cca tcc cct gtg cac agt ggc ttc ccc tcc ccg tcg gtg gcc      1488
   Thr Tyr Pro Ser Pro Val His Ser Gly Phe Pro Ser Pro Ser Val Ala
                   485                 490                 495
   acc acg tac tcc tct gtt ccc cct gct ttc ccg gcc cag gtc agc agc      1536
   Thr Thr Tyr Ser Ser Val Pro Pro Ala Phe Pro Ala Gln Val Ser Ser
               500                 505                 510
   ttc cct tcc tca gct gtc acc aac tcc ttc agc gcc tcc aca ggg ctt      1584
   Phe Pro Ser Ser Ala Val Thr Asn Ser Phe Ser Ala Ser Thr Gly Leu
           515                 520                 525
   tcg gac atg aca gca acc ttt tct ccc agg aca att gaa att tgc          1629
   Ser Asp Met Thr Ala Thr Phe Ser Pro Arg Thr Ile Glu Ile Cys
       530                 535                 540
<210> 4
   <211> 1629
   <212> DNA
   <213> Homo sapiens
<220>
   <221> CDS
   <222> (1) .. (1629)
   <223>
<400> 4
   atg gcc gcg gcc aag gcc gag atg cag ctg atg tcc ccg ctg cag atc       48
   Met Ala Ala Ala Lys Ala Glu Met Gln Leu Met Ser Pro Leu Gln Ile
   1               5                   10                  15
   tct gac ccg ttc gga tcc ttt cct cac tcg ccc acc atg gac aac tac       96
   Ser Asp Pro Phe Gly Ser Phe Pro His Ser Pro Thr Met Asp Asn Tyr
               20                  25                  30
   cct aag ctg gag gag atg atg ctg ctg agc aac ggg gct ccc cag ttc      144
   Pro Lys Leu Glu Glu Met Met Leu Leu Ser Asn Gly Ala Pro Gln Phe
           35                  40                  45
   ctc ggc gcc gcc ggg gcc cca gag ggc agc ggc agc aac agc agc agc      192
   Leu Gly Ala Ala Gly Ala Pro Glu Gly Ser Gly Ser Asn Ser Ser Ser
       50                  55                  60
   agc agc agc ggg ggc ggt gga ggc ggc ggg ggc ggc agc aac agc agc      240
   Ser Ser Ser Gly Gly Gly Gly Gly Gly Gly Gly Gly Ser Asn Ser Ser
   65                  70                  75                  80
   agc agc agc agc acc ttc aac cct cag gcg gac acg ggc gag cag ccc      288
   Ser Ser Ser Ser Thr Phe Asn Pro Gln Ala Asp Thr Gly Glu Gln Pro
                   85                  90                  95
   tac gag cac ctg acc gca gag tct ttt cct gac atc tct ctg aac aac      336
   Tyr Glu His Leu Thr Ala Glu Ser Phe Pro Asp Ile Ser Leu Asn Asn
               100                 105                 110
   gag aag gtg ctg gtg gag acc agt tac ccc agc caa acc act cga ctg      384
   Glu Lys Val Leu Val Glu Thr Ser Tyr Pro Ser Gln Thr Thr Arg Leu
           115                 120                 125
   ccc ccc atc acc tat act ggc cgc ttt tcc ctg gag cct gca ccc aac      432
   Pro Pro Ile Thr Tyr Thr Gly Arg Phe Ser Leu Glu Pro Ala Pro Asn
       130                 135                 140
   agt ggc aac acc ttg tgg ccc gag ccc ctc ttc agc ttg gtc agt ggc      480
   Ser Gly Asn Thr Leu Trp Pro Glu Pro Leu Phe Ser Leu Val Ser Gly
   145                 150                 155                 160
   cta gtg agc atg acc aac cca ccg gcc tcc tcg tcc tca gca cca tct      528
   Leu Val Ser Met Thr Asn Pro Pro Ala Ser Ser Ser Ser Ala Pro Ser
                   165                 170                 175
   cca gcg gcc tcc tcc gcc tcc gcc tcc cag agc cca ccc ctg agc tgc      576
   Pro Ala Ala Ser Ser Ala Ser Ala Ser Gln Ser Pro Pro Leu Ser Cys
               180                 185                 190
   gca gtg cca tcc aac gac agc agt ccc att tac tca gcg gca ccc acc      624
   Ala Val Pro Ser Asn Asp Ser Ser Pro Ile Tyr Ser Ala Ala Pro Thr
           195                 200                 205
   ttc ccc acg ccg aac act gac att ttc cct gag cca caa agc cag gcc      672
   Phe Pro Thr Pro Asn Thr Asp Ile Phe Pro Glu Pro Gln Ser Gln Ala
       210                 215                 220
   ttc ccg ggc tcg gca ggg aca gcg ctc cag tac ccg cct cct gcc tac      720
   Phe Pro Gly Ser Ala Gly Thr Ala Leu Gln Tyr Pro Pro Pro Ala Tyr
   225                 230                 235                 240
   cct gcc gcc aag ggt ggc ttc cag gtt ccc atg atc ccc gac tac ctg      768
   Pro Ala Ala Lys Gly Gly Phe Gln Val Pro Met Ile Pro Asp Tyr Leu
                   245                 250                 255
   ttt cca cag cag cag ggg gat ctg ggc ctg ggc acc cca gac cag aag      816
   Phe Pro Gln Gln Gln Gly Asp Leu Gly Leu Gly Thr Pro Asp Gln Lys
               260                 265                 270
   ccc ttc cag ggc ctg gag agc cgc acc cag cag cct tcg cta acc cct      864
   Pro Phe Gln Gly Leu Glu Ser Arg Thr Gln Gln Pro Ser Leu Thr Pro
           275                 280                 285
   ctg tct act att aag gcc ttt gcc act cag tcg ggc tcc cag gac ctg      912
   Leu Ser Thr Ile Lys Ala Phe Ala Thr Gln Ser Gly Ser Gln Asp Leu
       290                 295                 300
   aag gcc ctc aat acc agc tac cag tcc cag ctc atc aaa ccc agc cgc      960
   Lys Ala Leu Asn Thr Ser Tyr Gln Ser Gln Leu Ile Lys Pro Ser Arg
   305                 310                 315                 320
   atg cgc aag tac ccc aac cgg ccc agc aag acg ccc ccc cac gaa cgc      1008
   Met Arg Lys Tyr Pro Asn Arg Pro Ser Lys Thr Pro Pro His Glu Arg
                   325                 330                 335
   cct tac gct tgc cca gtg gag tcc tgt gat cgc cgc ttc tcc cgc tcc      1056
   Pro Tyr Ala Cys Pro Val Glu Ser Cys Asp Arg Arg Phe Ser Arg Ser
               340                 345                 350
   gac gag ctc acc cgc cac atc cgc atc cac aca ggc cag aag ccc ttc      1104
   Asp Glu Leu Thr Arg His Ile Arg Ile His Thr Gly Gln Lys Pro Phe
           355                 360                 365
   cag tgc cgc atc tgc atg cgc aac ttc agc cgc agc gac cac ctc acc      1152
   Gln Cys Arg Ile Cys Met Arg Asn Phe Ser Arg Ser Asp His Leu Thr
       370                 375                 380
   acc cac atc cgc acc cac aca ggc gaa aag ccc ttc gcc tgc gac atc      1200
   Thr His Ile Arg Thr His Thr Gly Glu Lys Pro Phe Ala Cys Asp Ile
   385                 390                 395                 400
   tgt gga aga aag ttt gcc agg agc gat gaa cgc aag agg cat acc aag      1248
   Cys Gly Arg Lys Phe Ala Arg Ser Asp Glu Arg Lys Arg His Thr Lys
                   405                 410                 415
   atc cac ttg cgg cag aag gac aag aaa gca gac aaa agt gtt gtg gcc      1296
   Ile His Leu Arg Gln Lys Asp Lys Lys Ala Asp Lys Ser Val Val Ala
               420                 425                 430
   tct tcg gcc acc tcc tct ctc tct tcc tac ccg tcc ccg gtt gct acc      1344
   Ser Ser Ala Thr Ser Ser Leu Ser Ser Tyr Pro Ser Pro Val Ala Thr
           435                 440                 445
   tct tac ccg tcc ccg gtt act acc tct tat cca tcc ccg gcc acc acc      1392
   Ser Tyr Pro Ser Pro Val Thr Thr Ser Tyr Pro Ser Pro Ala Thr Thr
       450                 455                 460
   tca tac cca tcc cct gtg ccc acc tcc ttc tcc tct ccc ggc tcc tcg      1440
   Ser Tyr Pro Ser Pro Val Pro Thr Ser Phe Ser Ser Pro Gly Ser Ser
   465                 470                 475                 480
   acc tac cca tcc cct gtg cac agt ggc ttc ccc tcc ccg tcg gtg gcc      1488
   Thr Tyr Pro Ser Pro Val His Ser Gly Phe Pro Ser Pro Ser Val Ala
                   485                 490                 495
   acc acg tac tcc tct gtt ccc cct gct ttc ccg gcc cag gtc agc agc      1536
   Thr Thr Tyr Ser Ser Val Pro Pro Ala Phe Pro Ala Gln Val Ser Ser
               500                 505                 510
   ttc cct tcc tca gct gtc acc aac tcc ttc agc gcc tcc aca ggg ctt      1584
   Phe Pro Ser Ser Ala Val Thr Asn Ser Phe Ser Ala Ser Thr Gly Leu
           515                 520                 525
   tcg gac atg aca gca acc ttt tct ccc agg aca att gaa att tgc          1629
   Ser Asp Met Thr Ala Thr Phe Ser Pro Arg Thr Ile Glu Ile Cys
       530                 535                 540
<210> 5
   <211> 9
   <212> DNA
   <213> Artificial
<220>
   <221> misc_feature
   <223> Oligonucleotide designed to act as decoy for Egr-1.
<400> 5
   gcgtgggcg 9
<210> 6
   <211> 9
   <212> DNA
   <213> Artificial
<220>
   <221> misc_feature
   <223> Oligonucleotide designed to act as decoy for Egr-1.
<400> 6
   gcgggggcg 9
<210> 7
   <211> 20
   <212> DNA
   <213> Artificial
<220>
   <221> misc_feature
   <223> Oligonucleotide designed to act as antisense DNA for Egr-1 mRNA.
<400> 7
   gcggggtgca ggggcacact 20
<210> 8
   <211> 30
   <212> DNA
   <213> Artificial
<220>
   <221> misc_feature
   <223> Oligonucleotide designed to act as primer for amplifying Egr-1 cDNA.
<400> 8
   ccgaattcag tgttccccgc gccccgcatg 30
<210> 9
   <211> 29
   <212> DNA
   <213> Artificial
<220>
   <221> misc_feature
   <223> Oligonucleotide designed to act as primer for amplifying Egr-1 cDNA.
<400> 9
   ggctcgagaa cctccatctg acctaagag 29
<210> 10
   <211> 26
   <212> DNA
   <213> Artificial
<220>
   <221> misc_feature
   <223> Oligonucleotide designed to act as sense strand of double-stranded DNA capable of binding with Egr-1.
<400> 10
   tgactcgccc tcgcccccgc gccggg 26
<210> 11
   <211> 26
   <212> DNA
   <213> Artificial
<220>
   <221> misc_feature
   <223> Oligonucleotide designed to act as sense strand of double-stranded DNA incapable of binding with Egr-1.
<400> 11
   tgactcgccc tcgacccagc gccggg 26

## Description

### Technical Field

The present invention relates to a method for screening a prophylactic and therapeutic product for renal diseases.

### Background Art

As a protein comprising the amino acid sequence represented by SEQ ID NO: I is known Early Growth Response-l (herein, abbreviated as Egr-1).

Egr-1 is activated by ischemia and is a transcription factor that induces the expression of an important regulator for inflammation, coagulation and vascular permeability (Nature Medicine, vol. 6, No. 12, p. 1355).

The international Application WO01/04356 discloses methods for diagnosing coronary artery disease associated with ischemia by measuring the induction of Egr-1 and early growth response factor-2 (Egr-2) and discloses screening methods for agents modulating Egr-1 or Egr-2 expression or activity.

Rupprecht et al. (Kidney International, 1997, vol. 51, p. 694-702) describes the increased expression of Egr-1 expression in a rat model of mesangioproliferative glomerulonephritis, in particular in glomerular mesangial cells. Furthermore, by adding Egr-1 antisense nucleotides to cultured mesangial cells, the authors showed that blocking Egr-1 expression inhibits the platelet-derived growth factor (PDGF)-induced growth of mesangial cells.

In addition, presently, as therapeutic products for renal diseases are used an angiotensin converting enzyme inhibitor, but the inhibitor has an effect on renal circulation kinetics, thereby being incapable of using for renal function highly impaired patients.

There is required a prophylactic and therapeutic product for a renal disease and the like, which have excellent effects and no adverse reactions, and a method for being able to screen such a prophylactic and therapeutic product.

### Disclosure of the Invention

The present inventors, as a result of earnest studies to solve the above-described problem, have for the first time found out that the expression of renal Egr-1 in a renal disease model animal is remarkably increased, and further for the first time that suppression of the expression of renal Egr-1 in a renal disease model animal can produce a therapeutic effect on a renal disease. The present inventors have further carried out studies based on findings thereof, thus leading to the completion of the present invention.

In other words, the present invention relates to:
1. A screening method for a prophylactic and therapeutic substance for a renal disease, which is characterized by:
   cultivating a cell that has the capability of producing a protein or salts thereof comprising an amino acid sequence represented by SEQ ID NO: 2 in the absence and the presence of a test compound,
   immobilizing on a solid phase a polynucleotide, to which the protein or salts thereof is capable of binding,
   adding to the solid phase the protein or salts thereof and an antibody against the protein or salts thereof; and
   measuring and comparing the production of the protein or salts thereof, and binding activity of the protein or salts thereof to the polynucleotide in the case where the cell is cultivated in the absence of the test compound and the case where the cell cultivated in the presence of the test compound.
2. The screening method according to paragraph 1, wherein the renal disease is an Egr-1 depending renal disease; and
   selecting a compound that decreases the amount of the protein or salts thereof and/or a compound that decreases the binding activity of the protein or salts thereof to the polynucleotide.
3. The screening method according to paragraph 1, wherein the renal disease is diabetic nephropathy.

### Best Mode for Carrying Out the Invention

In the present invention, the protein used in the methods claimed comprising the amino acid sequence represented by SEQ ID NO:2 and thus Egr-1 (hereinafter, may be abbreviated as a protein used in the present invention) may originate in cells (e.g., a liver cell, a retina cell, a nerve cell, a glial cell, at cell of pancreas, a bone marrow cell, a mesangial cell, a Langerhans' cell, an epidermic cell, an epithelial cell, a goblet cell, an endothelial cell, a smooth muscle cell, a fibroblast, a fibrocyte, a myocyte, a fat cell, immune cells (e.g., macrophage, a T cell, a B cell, a natural killer cell, a mast cell, neutrophil, basophil, eosinophil, monocyte), megakaryocyte, a synovial cell, a chondrocyte, a bone cell, an osteoblast, an osteoclast, a mammary gland cell, a hepatocyte or an interstitial cell, or corresponding precursor cells thereof, a stem cell, a cancer cell, etc.), or any tissues where such cells are present, e.g., brain or any regions of the brain (for example, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc. of warm-blooded animals (e.g., human, a mouse, a rat, a rabbit, a sheep, swine, bovine, a horse, a bird, a cat, a dog, a monkey, a chimpanzee, etc.). The protein may also be derived from a synthetic protein.

With proteins herein, the left end is the N terminal (the amino terminal) and the right end is the C terminal (the carboxyl terminal) in accordance with the conventional peptide marking. For the protein used in the present invention the C terminal may be any of a carboxyl group (-COOH), a carboxylate (-COO⁻), an amide (-CONH₂), and an ester (-COOR).

Here, R of the esters for use include, for example, C₁₋₆ alkyl groups such as methyl, ethyl, n-propyl, isopropyl, and n-butyl; C₃₋₈ cycloalkyl groups such as cyclopentyl and cyclohexyl; C₆₋₁₂ aryl groups such as phenyl and α-naphtyl; C₇₋₁₄ aralkyl groups such as phenyl-C₁₋₂ alkyl groups such as benzyl and phenethyl; and α-naphtyl-C₁₋₂ alkyl groups such as α-naphtylmethyl; a pivaloyloxymethyl group, and the like.

When the protein has a carboxyl group (or a carboxylate) in addition to the C terminal, a group in which the carboxyl group is subjected to amidation or esterification is also included in the protein used in the present invention. In this case, the esters for use include, for example, an ester of the above-mentioned C terminal, and the like.

Furthermore, the protein used in the present invention also includes proteins in which the amino groups (e.g., a methionine residue) of N terminals are protected by protecting groups (e.g.; C₁₋₆ acyl groups including C₁₋₆ alkanoyl groups such as a formyl group and an acetyl group; and the like), proteins made by subjecting glutamine residues of the N terminal, which are produced by cleavage in vivo, to pyroglutamine oxidation, proteins produced by protecting substituents (for example, -OH, -SH, an amino group, an imidazole group, an indole group, a guadinino group, and the like) on the side chains of intramolecular amino acids by suitable protecting groups (e.g.; C₁₋₆ acyl groups including C₁₋₆ alkanoyl groups such as a formyl group and an acetyl group; and the like), conjugated proteins such as a glycoprotein produced by bonding a sugar chain to a protein, and the like.

As salts of the protein used in the present invention, salts with physiologically acceptable acids (e.g., an inorganic acid and an organic acid), bases (e.g., alkaline metal salts) and the like are used, particularly physiologically acceptable acid addition salts are preferable. These salts for use include, for example, salts with inorganic acids (for example, hydrochloric acid, phosphoric acid, hydrobromic acid, sulfuric acid), salts with organic acids (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

The protein or salts thereof used in the present invention can be produced by publicly known protein purification methods from the above-mentioned cells or tissues of a warm-blooded animal. Specifically, the protein or salts thereof used in the present invention can be produced by a method that involves homogenizing tissues or cells of a warm-blooded animal, and then separating and purifying the soluble fraction by chromatography such as a reverse chromatography or an ion exchange chromatography.

The protein or salts thereof used in the present invention can also be produced in accordance with publicly known peptide synthesis processes.

The peptide synthesis processes may be any of, for example, a solid phase synthesis process and a liquid phase synthesis process. A target protein can be produced by a process that includes condensing a partial peptide or an amino acid capable of forming the protein used in the present invention with a remaining portion, and subsequently removing a protecting group when the resultant product has the protecting group.

Here, the condensation and the removal of the protecting group are conducted on the basis of publicly known methods, for example, including the methods indicated in [1] to [5] below:
[1] M. Bodanszky and M.A. Ondetti: Peptide Synthesis, Interscience Publishers, New York (1966);
[2] Schroeder and Luebke: The Peptide, Academic Press, New York (1965);
[3] Nobuo Izumiya, et al.: Peptide Gosei-no-Kiso to Jikken (Basics and experiments of peptide synthesis), published by Maruzen Co. (1975);
[4] Haruaki Yajima and Shunpei Sakakibara: Seikagaku Jikken Koza (Biochemical Experiment) 1, Tanpakushitsu no Kagaku (Chemistry of Proteins) IV, 205 (1977); and
[5] Haruaki Yajima, ed.: Zoku Iyakuhin no Kaihatsu (A sequel to Development of Pharmaceuticals), Vol. 14, Peptide Synthesis, published by Hirokawa Shoten.

A protein thus obtained can be purified and isolated by publicly known purification methods. Here, the purification methods include, for example, a solvent extraction, a column chromatography, a liquid chromatography, recrystallization, combinations thereof, and the like.

When a protein obtained by the above-mentioned methods is a free form, the free form can be converted into a suitable salt by publicly known methods or analogous methods thereto. Conversely, if a protein is obtained in the form of a salt, the salt can be converted into a free form or another salt by publicly known methods or analogous methods thereto.

The protein or salts thereof used in the present invention is normally synthesized using commercially available protein synthesizing resins. These resins include, for example, a chloromethyl resin, a hydroxymethyl resin, a benzhydrylamine resin, an aminomethyl resin, a 4-benzyloxybenzyl alcohol resin, a 4-methylbenzhydrylamine resin, a PAM resin, a 4-hydroxymethylmethylphenylacetamidomethyl resin, a polyacrylamide resin, a 4-(2',4'-dimethoxyphenyl-hydroxymethyl)phenoxy resin, a 4-(2',4'-dimethoxyphenyl-Fmoc aminoethyl)phenoxy resin, and the like.

Using such a resin, amino acids in which an α-amino group and a side chain functional group are appropriately protected are condensed on the resin according to the sequence of a desired protein based on publicly known condensing methods. Simultaneously with cleaving the protein from the resin at the end of reaction, various protecting groups are removed, and further the resultant protein is subjected to an intramoleclular disulfide bond-forming reaction in a highly diluted solution to yield a desired protein.

Condensation of the above-mentioned protected amino acids can utilize a variety of activating agents capable of being used for protein synthesis, but particularly carbodiimides are preferable. The carbodiimides for use include DCC, N,N'-diisopropylcarbodiimide, N-ethyl-N'-(3-dimethylaminopropyl)carbodiimide, and the like.

Condensation of protected amino acids is performed by, for example, directly adding the protected amino acids along with an activating agent and a racemization suppressing additive (e.g., HOBt and HOOBt) to the resin, or by adding the resin, after the protected amino acids are activated in advance to yield symmetric acid anhydrides or HOBt esters or HOOBt esters, to the resin.

Solvents used for activation of protected amino acids and condensation of protected amino acids with a resin are, as required, selected from solvents that are known to be capable of being used for protein condensation reactions. Examples of the solvents for use include acid amides such as N,N-dimethylformamide, N,N-dimethylacetamide, and N-methylpyrrolidone; halogenated hydrocarbons such as methylene chloride and chloroform; alcohols such as trifluoroethanol; sulfoxides such as dimethylsulfoxide; amines such as pyridine; ethers such as dioxane and tetrahydrofuran; nitriles such as acetonitrile and propionitrile; esters such as methyl acetate and ethyl acetate; suitable mixtures thereof; and the like.

The reaction temperature is, as necessary, selected from the range that is known to be used in protein condensation reactions, and normally is, as appropriate, from the range of about -20°C to about 50°C. An activated amino acid derivative is normally used from 1.5 to 4 times in excess. When the condensation is insufficient as the result of the test using a ninhydrin reaction, sufficient condensation can be carried out by repeating the condensation reaction without elimination of the protecting group. If the condensation is insufficient even though the condensation reaction is repeated, sufficient condensation can be conducted by acetylating unreacted amino acids using acetic anhydride or acetylimidazole.

A protecting method and a protecting group of a functional group that should not been involved in the reaction of raw materials, a method of removing the protecting group, a method of activating a functional group involved in the reaction, and the like can be, as required, selected from well-known groups or well-known means.

The protecting groups of an amino group of the amino acid starting materials include, for example, Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfonyl, diphenylphosphinothioyl, Fmoc, and the like.

A carboxyl group of the amino acid starting materials can be protected, for example, by alkyl esterification (e.g., linear, branched or cyclic alkyl esterification with methyl, ethyl, propyl, butyl, t-butyl, cyclopentyl, cyclohexyl, cycloheptyl, cyclooctyl, 2-adamantyl, and the like), aralkyl esterification (e.g., esterification with benzyl, 4-nitrobenzyl, 4-methoxybenzyl, 4-chlorobenzyl, benzhydryl), phenacyl esterification, benzyloxycarbonyl hydrazidation, t-butoxycarbonyl hydrazidation, trityl hydrazidation, or the like.

The hydroxyl group of serine can be protected by, for example, esterification or etherification. Suitable groups for esterification include, for example, lower (C₁₋₆) alkanoyl groups such as an acetyl group, aroyl groups such as a benzoyl group, and groups derived from carbonic acid such as benzyloxycarbonyl group, ethoxycarbonyl group, and the like. In addition, appropriate groups for etherification include, for example, a benzyl group, a tetrahydropyranyl group, a t-butyl group, and the like.

The protecting groups of the phenolic hydroxyl group of tyrosine include, for example, Bzl, Cl₂-Bzl, 2-nitrobenzyl, Br-Z, t-butyl, and the like.

The protecting groups of the imidazole of histidine include, for example, Tos, 4-methoxy-2,3,6-trimethylbenzenesulfonyl, DNP, benzyloxymethyl, Bum, Boc, Trt, Fmoc, and the like.

The methods of removing (eliminating) a protecting group include, for example, catalytic reduction under a flow of hydrogen gas in the presence of a catalyst such as Pd-black or Pd-carbon; acid treatment by means of anhydrous hydrogen fluoride, methanesulfonic acid, trifluoromethane-sulfonic acid, trifluoroacetic acid, or a mixture solution thereof; base treatment by means of diisopropylethylamine, triethylamine, piperidine or piperazine; and reduction with sodium in liquid ammonia. The elimination reaction by the above-described acid treatment is generally carried out at a temperature of from about -20°C to about 40°C. The acid treatment is efficiently conducted by adding a cation scavenger such as anisole, phenol, thioanisole, m-cresol, p-cresol, dimethylsulfide, 1,4-butanedithiol or 1,2-ethanedithiol. Also, a 2,4-dinitrophenyl group used as a protecting group of the imidazole of histidine is removed by thiophenol treatment. A formyl group used as a protecting group of the indole of tryptophan is removed by acid treatment in the presence of 1,2-ethanedithiol, 1,4-butanedithiol, or the like, as well as by alkali treatment of a dilute sodium hydroxide solution, dilute ammonia, or the like.

Those obtained by activation of carboxyl groups in the amino acid starting materials for use include, for example, a corresponding acid anhydride, an azide, an activated ester (an ester with an alcohol (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, or HOBt)). Those obtained by activation of amino groups in the amino acid starting materials for use include, for example, a corresponding phosphoric amide.

An amide of a protein can be produced, in accordance with publicly known methods, by amidation of the α-carboxyl group of the carboxy terminal amino acid of the protein.

An ester of a protein can be produced, for example, by condensing the α-carboxyl group of the carboxy terminal amino acid of the protein with a desired alcohol.

Furthermore, the protein or salts thereof used in the present invention can also be prepared by cultivating a transformant comprising the DNA encoding the protein used in the present invention, and subsequently separating and purifying the protein or salts thereof from the resultant culture.

DNA encoding the protein used in the present invention includes genomic DNA, a genomic DNA library, cDNA derived from the cells and tissues described above, cDNA libraries derived from the cells and tissues described above and synthetic DNA. A vector to be used for a library may be any of a bacteriophage, a plasmid, a cosmid, a phagemid, and the like. In addition, the DNA can also be amplified by reverse transcriptase polymerase chain reaction (hereinafter abbreviated as RT-PCR) using the total RNA or mRNA fractions prepared from the cells and tissues mentioned above.

DNA encoding the protein used in the present invention includes, for example, DNA comprising a base sequence represented by SEQ ID NO: 3 or SEQ ID NO: 4, and DNA which comprises a base sequence hybridizable to a base sequence represented by SEQ ID NO: 3 or SEQ ID NO: 4 under highly stringent conditions, and which encodes the above protein having substantially the same activity (e.g., a transcription controlling activity of a fibrosis relating gene, a cell cycle relating gene, or a thrombus relating gene, or the like) as that of a protein comprising the amino acid sequence represented by SEQ ID NO:2.

DNA, for use, hybridizable to a base sequence represented by SEQ ID NO: 3 or SEQ ID NO:4 under highly stringent conditions include, for example, DNA comprising a base sequence having at least about 50%, preferably at least about 60%, more preferably at least about 70%, more preferably at least about 80%, much more preferably at least about 90% and most preferably at least about 95% identity to the base sequence represented by SEQ ID NO: 3 or SEQ ID NO: 4, and the like.

Hybridization can be carried out by publicly known methods or analogous methods thereto, for example, in accordance with the method described in Molecular Cloning, 2nd (J. Sambrook et al., Cold Spring Harbor Lab. Press, 1989). Also, when a commercially available library is utilized, hybridization can be conducted according to the protocol described in the attached instructions. Preferably, hybridization can be carried out under highly stringent conditions.

The highly stringent conditions stand for, for example, those in a sodium concentration of about 19 to about 40 mM, preferably about 19 to about 20 mM at a temperature of about 50°C to about 70°C, preferably about 60°C to about 65°C. In particular, the cases where a sodium concentration is about 19 mM and where the temperature is about 65°C are preferable.

DNA encoding a protein comprising the amino acid sequence represented by SEQ ID NO: 2 includes DVA comprising the amino acid sequence represented by SEQ DD NO: 3 or SEQ ID NO: 4.

DNA completely encoding the protein used in the present invention can be cloned by amplification by means of a PCR method using a synthetic DNA primer having a part of the base sequence encoding the protein used in the present invention, or hybridization of DNA inserted into an appropriate expression vector with a DNA fragment encoding a part or entire region of the protein used in the present invention, or with a labeled, synthetic DNA fragment. Hybridization can be carried out, for example, in accordance with the method described in Molecular Cloning, 2nd, J. Sambrook et al., Cold Spring Harbor Lab. Press, 1989 and the like. Also, when a commercially available library is employed, hybridization can be performed according to the protocol described in the attached instructions.

Use of publicly known kits such as Mutan^{™}-super Express Km (Takara Shuzo Co., Ltd.), Mutan^{™}-K (Takara Shuzo Co., Ltd.), and the like, can convert a base sequence of DNA in accordance with publicly known methods such as the ODA-LA PCR method, the Gapped duplex method or the Kunkel method, or analogous methods thereto.

DNA encoding a cloned protein can be used, depending on purposes, directly or, if desired, after digestion with a restriction enzyme or after addition of a linker thereto. The DNA has ATG as a translation initiation codon on the 5' terminal side, and may have TAA, TGA or TAG as a translation termination codon on the 3' terminal side. These translation initiation and termination codons can be added by means of an appropriate synthetic DNA adapter.

The aforementioned expression vector can be produced, for example, by excising a target DNA fragment from DNA encoding the protein used in the present invention, and linking the DNA fragment to the downstream of a promoter in an appropriate expression vector.

The expression vectors for use include a plasmid derived from E. coli (e.g., pBR322, pBR325, pUC12, or pUC13); a plasmid derived from Bacillus subtilis (e.g., pUB110, pTP5, or pC194); a plasmid derived from yeast (e.g., pSH19, or pSH15), a bacteriophage such as λ phage; animal viruses such as a retrovirus, a vaccinia virus, or a baculovirus; pA1-11, pXT1, pRc/CMV, pRc/RSV, pcDNAI/Neo, and the like.

The promoter may be any promoter suitable with a host to be used for gene expression.

For instance, when the host is an animal cell, the promoters for use include an SRα promoter, an SV40 promoter, an LTR promoter, a CMV (Cytomegalovirus) promoter, an HSV-TK promoter, and the like. Of these, a CMV promoter, an SRα promoter, and the like are preferable.

If the host is a bacterium of the genus Escherichia, the preferred promoters include a trp promoter, a lac promoter, a recA promoter, a λP_{L} promoter, an lpp promoter, a T7 promoter, and the like.

In the case of using a bacterium of the genus Bacillus as the host, the preferred promoters include an SPO1 promoter, an SPO2 promoter and a penP promoter and the like.

When yeast is used as the host, the preferred promoters include a PHOS promoter, a PGK promoter, a GAP promoter, ADH promoter, and the like.

Where the host is an insect cell, the preferred promoters include a polyhedrin prompter, a P10 promoter, and the like.

The expression vector, in addition to the above, can optionally utilize an expression vector that comprises an enhancer, a splicing signal, a polyA addition signal, a selection marker, an SV40 replication origin (hereinafter may be abbreviated as SV40ori), and the like. The selection markers include, for example, a dihydrofolate reductase (hereinafter may be abbreviated as dhfr) gene [methotrexate (MTX) resistance], an ampicillin resistant gene (hereinafter may be abbreviated as Amp^{r}), a neomycin resistant gene (hereinafter may be abbreviated as Neo^{r}, G418 resistance), and the like. In particular, when a dhfr gene defective Chinese hamster cell is used and when a dhfr gene is used as a selection marker, a target gene can also be selected by a thymidine free medium.

In addition, as required, a signal sequence that matches with a host is added to the N terminal of the protein used in the present invention. When the host is a bacterium of the genus Escherichia, a PhoA signal sequence, an OmpA signal sequence, and the like are utilized. When the host is a bacterium of the genus Bacillus, an α-amylase signal sequence, a subtilisin signal sequence, and the like are used. When the host is yeast, an MFα signal sequence, a SUC2 signal sequence, and the like are used. When the host is an animal cell, an insulin signal sequence, an α-interferon signal sequence, an antibody molecule signal sequence, and the like are employed.

A transformant comprising DNA encoding the protein used in the present invention can be prepared in accordance with publicly known methods by transforming the host using an expression vector comprising the DNA.

Here, the expression vectors include the above-mentioned expression vectors.

The hosts for use include, for example, a bacterium of the genus Escherichia, a bacterium of the genus Bacillus, yeast, an insect cell, an insect, an animal cell, and the like.

The bacteria of the genus Escherichia, which are used, include, for example, Escherichia coli K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

The bacteria of the genus Bacillus for use include, for example, Bacillus subtilis MI114 (Gene, 24, 255 (1983)), 207-21 (Journal of Biochemistry, 95, 87 (1984)), etc.

The yeast for use includes, for example, Saccharomyces cereviseae AH22, AH22R⁻, NA87-11A, DKD-5D, 20B-12, Schizosaccharomyces pombe NCYC1913, NCYC2036, Pichia pastoris KM71, and the like.

The insect cells for use include, for example, a cell in which the virus is AcNPV, a Spodoptera frugiperda cell (a Sf cell), a MG1 cell derived from the mid-intestine of Trichoplusia ni, a High Five^{™} cell derived from an egg of Trichoplusia ni, a cell derived from Mamestra brassicae, a cell derived from Estigmena acrea, and the like. With the virus BmNPV, a Bombyx mori N cell (a BmN cell), and the like are used as insect cells. The Sf cells for use include, for example, a Sf9 cell (ATCC CRL1711), a Sf21 cell (both in Vaughn, J. L. et al., In Vivo, 13, 213-217 (1977), and the like.

The insects for use include, for example, a larva of Bombyx mori (Maeda, et al., Nature, 315, 592 (1985)), and the like.

The animal cells for use include, for example, a monkey cell COS-7, Vero, a Chinese hamster cell CHO (hereafter abbreviated as a CHO cell), a dhfr gene defective Chinese hamster cell CHO (hereafter abbreviated as a CHO(dhfr⁻) cell), a mouse L cell, mouse AtT-20, a mouse myeloma cell, rat GH3, a human FL cell, and the like.

Transformation can be carried out according to the kinds of hosts in accordance with publicly known methods.

A bacterium of the genus Escherichia can be transformed, for example, in accordance with a method described in Proc. Natl. Acad. Sci. U.S.A., 69, 2110 (1972), Gene, 17, 107 (1982), etc.

A bacterium of the genus Bacillus can be transformed, for example, according to a method described in Molecular and General Genetics, 168, 111 (1979), etc.

Yeast can be transformed, for example, in accordance with a method described in Methods in Enzymology, 194, 182-187 (1991), Proc. Natl. Acad. Sci. U.S.A., 75, 1929 (1978), etc.

An insect cell and an insect can be transformed, for example, according to a method described in Bio/Technology, 6, 47-55(1988), etc.

An animal cell can be transformed, for example, in accordance with a method described in Saibo Kogaku (Cell Engineering), extra issue 8, Shin Saibo Kogaku Jikken Protocol (New Cell Engineering Experimental Protocol), 263-267 (1995), published by Shujunsha, or Virology, 52, 456 (1973).

Cultivation of a transformant can be carried out according to the kind of hosts in accordance with publicly known methods.

For instance, when a transformant whose host is a bacterium of the genus Escherichia or the genus Bacillus, a medium for cultivation is preferably a liquid medium. Also, the medium preferably comprises a carbon source, a nitrogen source, an inorganic material, and the like, necessary for the growth of the transformant. Here, the carbon sources include, for example, glucose, dextrin, soluble starch, sucrose, and the like. The nitrogen sources include, for example, inorganic and organic materials such as an ammonium salt, a nitrate salt, corn steep liquor, peptone, casein, meat extract, soybean cake, potato extract, and the like. The inorganic materials include, for example, calcium chloride, sodium dihydrogenphosphate, magnesium chloride, and the like. In addition, to the medium may also be added yeast extract, vitamins, growth promoting factor, and the like. Preferably, the pH of the medium is from about 5 to about 8.

The media in the case where a transformant whose host is a bacterium of the genus Escherichia is cultivated preferably include, for example, a M9 medium supplemented with glucose and a Casamino acid (Miller, Journal of Experiments in Molecular Genetics, 431-433, Cold Spring Harbor Laboratory, New York, 1972). As required, in order to efficiently activate a promoter, a chemical such as 3β-indolylacrylic acid may also be added to the medium.

Cultivation of a transformant whose host is a bacterium of the genus Escherichia is normally carried out at about 15°C to about 43°C for about 3 to about 24 hours. As necessary, the culture may be aerated or agitated.

Cultivation of a transformant whose host is a bacterium of the genus Bacillus is normally carried out at about 30°C to about 40°C for about 6 to about 24 hours. As necessary, the culture may be aerated or agitated.

The media in the case where a transformant whose host is yeast is cultivated include, for example, a minimal medium of Burkholder (Bostian, K. L. et al., Proc. Natl. Acad. Sci. U.S.A., 77, 4505 (1980)), a SD medium comprising a 0.5% Casamino acid (Bitter, G. A. et al., Proc. Natl. Acad. Sci. U.S.A., 81, 5330 (1984)), etc. Preferably, the pH of the medium is from about 5 to about 8. Cultivation is normally conducted at about 20°C to about 35°C for about 24 to about 72 hours. As needed, the culture may be aerated or agitated.

The media for use in the case where a transformant whose host is an insect cell or an insect is cultivated include, for example, Grace's Insect Medium (Grace, T. C. C., Nature, 195, 788 (1962)) to which an additive such as immobilized 10% bovine serum is added, as appropriate, and the like. Preferably, the pH of the medium is from about 6.2 to about 6.4. Cultivation is normally carried out at about 27°C for about 3 to about 5 days. As needed, the culture may be aerated or agitated.

The media for use in the case where a transformant whose host is an animal cell is cultivated include, for example, a MEM medium containing about 5 to about 20% of a fetal bovine serum (Science, 122, 501 (1952)), a DMEM medium (Virology, Vol. 8, 396 (1959)), a RPMI 1640 medium (The Journal of the American Medical Association, 199, 519 (1967)), a 199 medium (Proceeding of the Society for the Biological Medicine, 73, 1 (1950)), etc. Preferably, the pH of the medium is from about 6 to about 8. Cultivation is normally carried out at about 30°C to about 40°C for about 15 to about 60 hours. As required, the culture may be aerated or agitated.

As discussed so far, the protein used in the present invention can be produced in a cell, in a cell membrane, or outside a cell, of a transformant.

The protein used in the present invention can be separated and purified from a culture obtained by cultivating the transformant in accordance with publicly known methods.

For example, when the protein used in the present invention is extracted from a cultivated bacterium or cell, a method which involves suspending a bacterium or a cell collected from a culture by publicly known methods in an appropriate buffer, disrupting the bacterium or the cell by ultrasonication, lysozyme and/or freeze-thaw cycling, and then obtaining a crude extract by centrifugation, or filtration; or the like is used, as appropriate. The buffer may contain a protein modifier such as urea or guanidine hydrochloride, or a surfactant such as Triton X-100^{™}, and the like. Additionally, extraction of the protein used in the present invention from a nucleus fraction is conducted by a method that includes treating the precipitate obtained by the aforementioned centrifugation, or filtration with hypertonic solution or the like, and subsequently collecting the supernatant by centrifugation to thereby obtain a crude extract of the nucleus protein, or a similar method. When the protein is secreted in the culture, a method is used wherein the culture supernatant is collected from the culture by publicly known methods.

The culture supernatant or the protein contained in extract thus obtained can be separated and purified in accordance with publicly known methods. Such methods for use include a method that utilizes solubility such as salting out, or solvent precipitation; a method that utilizes mainly difference in molecular weight such as dialysis, ultrafiltration, gel filtration, SDS-polyacrylamide gel electrophoresis, or the like; a method that utilizes difference in electric charge such as ion exchange chromatography, or the like; a method that utilizes specific affinity such as affinity chromatography, or the like; a method that utilizes difference in hydrophobicity such as reverse phase high performance liquid chromatography, or the like; a method that utilizes difference in isoelectric point such as isoelectrofocusing electrophoresis; and the like. These methods can be, as appropriate, used in combination as well.

When the protein thus obtained is in a free form, it can be converted into a salt by publicly known methods or analogous methods thereto; if the protein is obtained in the form of a salt, it can be convened into a free form or in the form of a different salt by publicly known methods or analogous methods thereto.

Moreover, the protein produced by the transformant can also arbitrarily be modified by the action of an appropriate protein-modifying enzyme, prior to or subsequent to the purification, or a polypeptide thereof can also be removed partially. The protein-modifying enzymes for use include, for example, trypsin, chymotrypsin, arginyl endopeptidase, protein kinase, glycosidase, and the like.

The presence of the thus produced protein used in the present invention can be confirmed by an enzyme immunoassay using a specific antibody, the western blotting technique or the like.

Diseases associated with the protein used in the present invention are preferably Egr-1 depending diseases that develop depending on the amount of Egr-1 in the body.

Diseases associated with the protein of the present invention are renal diseases, preferably Egr-1 depending diseases. In particular, renal Egr-1 depending renal diseases are preferable.

The present invention relates to a screening method for a prophylactic and therapeutic substance for a renal disease which is characterized by using the protein used in the present invention.

The screening method of the present invention is carried out, by 1) comparing the amount of production of the protein used in the present invention in the case where a cell having the capability of producing the protein used in the present invention is cultivated with that of production of the protein used in the present invention in the case where a cell having the capability of producing the protein used in the present invention is cultivated in the presence of a test compound; 2) comparing the activity of the protein used in the present invention in the case where a cell having the capability of producing the protein used in the present invention is cultivated with that of the protein used in the present invention in the case where a cell having the capability of producing the protein used in the present invention is cultivated in the presence of a test compound; 3) comparing the activity of the protein used in the present invention in the presence of and in the absence of a test compound; and the like.

The cell having the capability of producing the protein used in the present invention is not limited as long as it is a cell having the capability of producing the protein, but a cell is preferable wherein production of the protein (Egr-1) used in the present invention is derived depending on a variety of stimuli such as an oxidation stress and a proliferation factor (hereafter, may comprehensively be referred to as "Egr-1 expression deriving factor") treatment.

The cell having the capability of producing the protein used in the present invention may also be the aforementioned "transformant comprising DNA encoding the protein of the present invention." Preferable examples of the cell having the capability of producing the protein used in the present invention include, for example, a cell isolated from a kidney of a mammal (preferably, a human, a rat, a mouse, or the like). These cells may also be immortalized cells.

The cell having the capability of producing the protein used in the present invention is cultivated as for the aforementioned transformant.

The test compounds include, for example, a peptide, a protein, a non-peptidic compound, a synthetic compound, a fermented product, a cell extract, a plant extract, an animal tissue extract, and the like.

The protein used in the present invention can be quantitatively determined by publicly known methods, for example, methods using an antibody to the protein used in the present invention such as western analysis and the ELISA method, or analogous methods thereto.

The antibody to the protein used in the present invention may be any of monoclonal and polyclonal antibodies as long as it is an antibody that can recognize the protein used in the present invention. Additionally, the antibody may be an antibody molecule itself, and F(ab')₂, Fab', or Fab fraction of the antibody molecule. Also, an antibody may be labeled as well.

The labeling agent used for labeling an antibody includes, for example, a radioisotope, an enzyme, a fluorescent substance, a luminescent substance, and the like. The radioisotope for use include, for example, [¹²⁵I], [¹³¹I], [³H], [¹⁴C], and the like. The enzyme for use is preferably stable and high in specific activity, and includes, for example, β-galactosidase, β-glucosidase, alkaline phosphatase, peroxidase, malate dehydrogenase, and the like. The fluorescent substance for use includes, for example, fluorescamine, fluorescein isothiocyanate, and the like. The luminescent substance for use includes, for example, luminol, a luminol derivative, luciferin, lucigenin, and the like. Furthermore, the biotin-avidin system can also be used for binding an antibody or an antigen to the labeling agent.

When the protein used in the present invention is quantified, a protein to be quantified may be any of proteins contained in a cell or secreted to the outside of a cell, or the total of both also.

In addition, where the protein used in the present invention contained in a cell is quantified, it is preferably quantified after the cell is treated with a suitable immobilizing solution or a membrane permeation promoting agent. Moreover, after the cell is suspended in an appropriate buffer and destroyed with a supersonic wave or by freeze melt, the protein in the crashed solution can be determined as well. As required, after the protein in the crashed solution is separated and purified, the protein may also be quantified.

The activity of the protein used in the screening method of the present invention is a "binding activity to the polynucleotide, to which the protein used in the present invention is capable of binding".

The polynucleotide includes, for example, a polynucleotide comprising a base sequence represented by SEQ ID NO: 5 or SEQ ID NO: 6 described below (preferably, a polynucleotide having a base sequence represented by SEQ ID NO: 5 or SEQ ID NO: 6), and the like.

The above-mentioned binding activity can be measured by publicly known methods, e.g., the method of Shi-Fang Yan, et al. (Proc. Natl. Acad. Sci. U.S.A. 95, 8298-8303, 1998) or an analogous method thereto using electrophoretic mobility shift assay. Or, the activity can also be measured by immobilizing on a suitable solid phase (e.g., a microtighter plate, and the like) the above polynucleotide to which the protein used in the present invention is capable of binding (e.g., contacting the biotin-labeled polynucleotide with a (strepto)avidin-immobilized solid phase; or the like), adding to the solid phase the protein (or a fraction comprising it) used in the present invention and a labeled antibody to the protein used in the present invention (or an antibody to the protein used in the present invention and a secondary labeled antibody for the antibody) and reacting them, and then quantifying the protein used in the present invention bonded to the solid phase in accordance with the methods such as an ELISA method and the like or analogous methods thereto. Here, when the protein used in the present invention is provided by a culture obtained by cultivating a cell having the capability of producing the protein used in the present invention, the amount of production of the protein used in the present invention in the cell can be determined at the same time. In addition, a cell can be cultivated, as necessary, under the stimulus of an Egr-1 expression deriving factor.

The above-described screening method makes it possible to screen a prophylactic and therapeutic substance for renal diseases associated with the protein used in the present invention, and thus a compound regulating (promoting or inhibiting) the production of the protein used in the present invention, or a compound resulting (promoting or inhibiting) the activity of the protein used in the present invention.

For instance, a test compound that decreases the amount of the protein used in the present invention by about 20% or more, preferably by about 30% or more, and more preferably by about 50% or more, can be selected as a compound for inhibiting the production of the protein used in the present invention.

A test compound that decreases the binding activity of the protein used in the present invention by about 20% or more, preferably by about 30% or more, and more preferably by about 50% or more, can be selected as a compound for inhibiting the binding activity of the protein used in the present invention.

Furthermore, in the above-described screening method, in place of comparing the amount of production of the protein used in the present invention in the presence of and in the absence of a test compound, when the quantification and comparison of the amount of the protein of a cell or another test organism derived from an animal that is possibly infected by a disease associated with the protein or salts thereof used in the present invention and that of the protein used in the present invention in a cell or another test organism of a normal control animal leads to the confirmation of an increase or decrease in the amount of the protein in the test organism, it is possible to diagnose the test organism being infected by a disease associated with the protein used in the present invention, or the test organism being to be highly possibly infected by the disease.

Moreover, similarly, if the quantification and comparison of the activity of the protein used in the present invention results in the confirmation of an increase or decrease in the activity of the protein in the test organism, it is possible to diagnose the test organism being infected by a disease associated with the protein used in the present invention, or the test organism being to be highly possibly infected by the disease.

The prophylactic and therapeutic substance for renal diseases, obtained by utilizing the screening method of the present invention, may be any of a peptide, a protein, a non-peptide compound, a synthetic compound, a fermented product, a cell extract, a plant extract, an animal tissue extract, plasma, and the like. These may form salts, and examples of the salts include similar salts to the salts of the protein used in the present invention discussed above.

The "prophylactic and therapeutic substance (compound) for renal diseases, obtained by the screening method of the present invention, can be used as a prophylactic and therapeutic product for renal diseases after processing into a pharmaceutical composition by admixing, as required, with a pharmacologically acceptable carrier.

Here, examples of the pharmacologically acceptable carrier include a variety of organic and inorganic carrier substances conventionally used as preparation materials, and these are formulated as excipients, lubricants, binders, and disintegrators, in solid preparations; as solvents, solubilizing agents, suspending agents, isotonizing agents, buffering agents, and soothing agents, in liquid preparations, and the like. Also, as necessary, preparation additives such as antiseptics, antioxidants, coloring agents, sweeteners, and the like can be employed.

The excipients suitably include, for example, lactose, saccharose, D-mannitol, D-sorbitol, starch, gelatinized starch, dextrin, crystalline cellulose, low substituted hydroxypropyl cellulose, sodium carboxymethyl cellulose, gum arabic, dextrin, pullulan, light silicic acid anhydride, synthetic aluminum silicate, magnesium metasilicate aluminate, and the like.

Examples of the suitable lubricants include magnesium stearate, calcium stearate, talc, colloidal silica, and the like.

Examples of the suitable binders include gelatinized starch, sucrose, gelatin, gum arabic, methyl cellulose, carboxymethyl cellulose, sodium carboxymethyl cellulose, crystalline cellulose, saccharose, D-mannitol, trehalose, dextrin, pullulan, hydroxypropyl cellulose, hydroxypropylmethyl cellulose, polyvinyl pyrrolidone, and the like.

Examples of the suitable disintegrators include lactose, saccharose, starch, carboxymethyl cellulose, calcium carboxymethyl cellulose, sodium crosscarmellose, sodium carboxymethyl starch, light silicic acid anhydride, low substituted hydroxypropyl cellulose, and the like.

Examples of the suitable solvents include injection water, physiological saline, Ringer's solutions, alcohol, propylene glycol, polyethylene glycol, sesame oil, corn oil, olive oil, cottonseed oil, and the like.

Examples of the suitable solubilizing agents include polyethylene glycol, propylene glycol, D-mannitol, trehalose, benzyl benzoate, ethanol, trisaminomethane, cholesterol, triethanol amine, sodium carbonate, sodium citrate, sodium salicylate, sodium acetate, and the like.

Examples of the suitable suspending agents include surfactants such as stearyl triethanol amine, sodium lauryl sulfate, lauryl aminopropionic acid, lecithin, benzalkonium chloride, benzethonium chloride, and glycerin monostearate; e.g., hydrophilic polymers such as polyvinyl alcohol, polyvinyl pyrrolidone, sodium carboxymethyl cellulose, methyl cellulose, hydroxymethyl cellulose, hydroxyethyl cellulose, and hydroxypropyl cellulose; Polysorbates, polyoxyethylene hardened castor oil, and the like.

Examples of the suitable isotonizing agents include sodium chloride, glycerin, D-mannitol, D-sorbitol, glucose, and the like.

Examples of the suitable buffering agents include buffers of a phosphate, an acetate, a carbonate, a citrate, and the like.

Examples of the suitable soothing agents include benzyl alcohol and the like.

Examples of the suitable antiseptics include a paraoxybenzoate, chlorobutanol, benzyl alcohol, phenthyl alcohol, dehydroacetic acid, sorbic acid, and the like.

Examples of the suitable antioxidants include a sulfide, an ascorbate, and the like.

Examples of the suitable coloring agents include water-soluble, edible tar pigments (e.g., edible pigments such as Edible Red Nos.2 and 3, Edible Yellow Nos. 4 and 5, and Edible Blue Nos. 1 and 2, water-insoluble lake pigments (e.g., an aluminum salt of the aforementioned water-soluble, edible tar pigment, and the like), natural pigments (β-carotene, chlorophyll, red iron oxide, and the like).

Examples of the suitable sweeteners include sodium saccharate, dipotassium glycyrrhizinate, aspartame, stevia, and the like.

Dosage forms of the aforementioned pharmaceutical composition include oral agents such as tablets, capsules (including soft capsules and microcapsules), granules, powders, syrups, emulsions, and suspensions; non-oral agents such as injections (e.g., subcutaneous injections, intravenous injections, intramuscular injections, intraperitoneal injections, and the like), external agents (e.g., nasal preparations, transdermal preparations, ointments, and the like), suppositories (e.g., rectal suppositories, vaginal suppositories, and the like), pellets, drip infusions, sustained-release preparations (e.g., sustained-release microcapsules, and the like), etc. These are safely administered orally or non-orally.

The pharmaceutical composition can be produced by a conventional method in the preparation technique field, for example, by a method described in Japanese Pharmacopoeia, or the like. Hereinafter, a method for specifically producing a preparation will be set forth in detail. The content of the compound in the pharmaceutical composition, obtained by the screening method of the present invention, varies depending on the dosage form, the dose of the compound, etc.; and it is for example from about 0.1 to 100% by weight.

For instance, an oral agent is produced by adding to an effective ingredient an excipient (e.g., lactose, saccharose, starch, D-mannitol, or the like), a disintegrator (e.g., calcium carboxymethyl cellulose, or the like), a binder (e.g., gelatinized starch, gum arabic, carboxymethyl cellulose, hydroxypropyl cellulose, polyvinyl pyrrolidone, or the like), a lubricant (e.g., talc, magnesium stearate, polyethylene glycol 6000, or the like), etc., compression molding the resultant mixture, and subsequently, as required, coating the resulting material with a coating base by a publicly known method for the purpose of masking of a taste, solubility in intestines or durability.

The coating base includes, for example, a sugar-coating base, a water-soluble film coating base, an enteric film coating base, a sustained-release film coating base, and the like.

The sugar-coating bases for use includes saccharose, and further may include one species or two or more species in combination selected from the group consisting of talc, precipitated calcium carbonate, gelatin, gum arabic pullulan, carnauba wax, and the like.

The water-soluble film coating base includes, for example, cellulose base polymers such as hydroxypropyl cellulose, hydroxypropylmethyl cellulose, hydroxyethyl cellulose, and methylhydroxyethyl cellulose; synthetic polymers such as polyvinylacetal diethylanimoacetate, aminoalkylmethacrylate copolymer E (Eudragit-E (trade name), Rohm Pharma Corp.), and polyvinyl pyrrolidone; polysaccharides such as pullulan; and the like.

The enteric film coating base includes, for example, cellulose base polymers such as hydroxypropylmethyl cellulose phthalate, hydroxypropylmethyl cellulose acetate succinate, carboxymethylethyl cellulose, and cellulose acetate phthalate; acrylic acid base polymers such as Methacrylic Acid Copolymer L (Eudragit-L (trade name), Rohm Pharma Corp.), Methacrylic Acid Copolymer LD (Eudragit-L-30D55 (trade name), Rohm Pharma Corp.), and Methacrylic Acid Copolymer S (Eudragit-S (trade name), Rohm Pharma Corp.); natural substances such as shellac.

The sustained-release film coating base includes, for example, cellulose base polymers such as ethyl cellulose; acrylic acid base polymers such as Aminoalkyl Methacrylate Copolymer RS (Eudragit-RS (trade name), Rohm Pharma Corp.), and an ethyl acrylate-methylmethacrylate coplymer suspension (Eudragit-NE (trade name), Rohm Pharma Corp.); and the like.

The above-mentioned coating bases may also be used in a mixture of two or more species thereof in a suitable ratio. Also, when coating, for example, sunproofing agents such as titanium oxide and diiron trioxide may be used as well.

An injection is produced by dissolving, suspending or emulsifying an effective ingredient in an aqueous solvent (e.g., distilled water, physiological saline, Ringer's solution, and the like), an oily solvent (e.g., vegetable oils such as olive oil, sesame oil, cottonseed oil, corn oil, propylene glycol, or the like), or the like, together with a dispersing agent (e.g., polysorbate 80, polyoxyethylene hardened castor oil, or the like, polyethylene glycol, carboxymethyl cellulose, sodium alginate, or the like), a preservative (e.g., methylparaben, propylparaben, benzyl alcohol, chlorobutanol, phenol, or the like), an isotonizing agent (e.g., sodium chloride, glycerin, D-mannitol, D-sorbitol, glucose, or the like), or the like. At this time, if desired, additives such as a solubilizing agent (e.g., sodium salicylate, sodium acetate, or the like), a stabilizer (e.g., human serum alubumin, or the like), a soothing agent (e.g., benzyl alcohol, or the like), and the like may also be used. An injection solution is normally packed in a suitable ampule.

A preparation thus obtained is safe and low in toxicity, and thus can be orally or non-orally administered to, for example, mammals (e.g., a human, a mouse, a rat, a rabbit, a sheep, a pig, a bovine, a horse, a cat, a dog, a monkey, a chimpanzee, etc.).

The dose of a prophylactic and therapeutic product for diseases associated with the protein used in the present invention varies depending on a target disease, a target to be administered, a route for administration, or the like. For example, with an adult patient (body weight : 60 kg) suffering from a renal disease, the dose is from about 0.1 to about 100 mg, preferably from about 1.0 to about 50 mg, more preferably from about 1.0 to about 20 mg, per day as an effective ingredient, i.e., a compound obtained by the screening method of the present invention.

An antibody to the protein used in the present invention can be produced in accordance with a method of producing publicly known antibody or antiserum using the protein as an antibody. A monoclonal antibody or a polyclonal antibody to the protein used in the present invention can be produced, for example, as explained below.

### [Preparation of Monoclonal Antibody]

### (a) Preparation of Monoclonal Antibody-producing Cell

The protein used in the present invention is administered to a warm-blooded animal either solely or along with a carrier or a diluent to a site where the administration makes it possible to produce an antibody. In order to enhance antibody productivity when administration, a complete Freund's adjuvant or an incomplete Freund's adjuvant may be administered. The administration is normally performed once in two to six weeks and 2 to 10 times in total. The warm-blooded animals to be used include, for example, mammals (or a chicken and the like besides mammals) such as a monkey, a rabbit, a dog, a guinea pig, a mouse, a rat, a sheep, a goat, and the like, with a mouse and a rat being preferably used.

For instance, a monoclonal antibody-producing hybridoma can be prepared by a method that involves selecting a warm-blooded animal immunized with an antigen, e.g., a mouse that has indicated the antibody titer, collecting the spleen or lymph node two to five days after the final immunization, and then fusing an antibody-producing cell contained therein with a myeloma cell of the same or different animal species. The antibody titer in an antiserum is measured, for example, by reacting the labeled protein to be described below with the antiserum and then determining the activity of the labeling agent bound to the antibody. The fusing operation can be conducted according to publicly known methods, for example, the method of Koehler and Milstein (Nature, 256, 495, 1975). The fusing promoters include, for example, polyethylene glycol (PEG), Sendai virus, and the like, with PEG being preferably used.

The myeloma cell includes, for example, that of a warm-blooded animal such as NS-1, P3U1, SP2/0, AP-1, and the like, with P3U1 being preferably used. A preferred ratio of the number of antibody-producing cells to be used (spleen cells) to that of myeloma cells is from about 1:1 to about 20:1; PEG (preferably PEG 1000 to PEG 6000) is added in a concentration of from about 10 to about 80%, followed by incubating at about 20 to about 40°C, preferably at about 30 to about 37°C for about 1 to about 10 minutes, thereby being capable of efficiently fusing the cells.

A monoclonal antibody-producing hybridoma can be screened, for example, by a method that include adding the supernatant of a hybridoma culture to a solid phase (e.g., microplate) adsorbed with a protein antigen directly or together with a carrier, adding an anti-immunoglobulin antibody (when a mouse cell is used for cell fusion, an anti-mouse immunoglobulin antibody is used) labeled with a radioactive substance or an enzyme, or Protein A, and then detecting the monoclonal antibody bound to the solid phase; by a method that involves adding the supernatant of a hybridoma culture to a solid phase adsorbed with an anti-immunoglobulin antibody or Protein A, adding a protein labeled with a radioactive substance or an enzyme, and subsequently detecting the monoclonal antibody bound to the solid phase; or by a similar method.

A monoclonal antibody can be selected by publicly known methods or by analogous methods thereto. Selection of a monoclonal antibody can be normally carried out in a medium for animal cells to which HAT (hypoxanthine, aminopterin and thymidine) is added. The medium for selection and growth of a monoclonal antibody may be any medium as long as a hybridoma can grow therein. The medium to be used includes, for example, a RPMI 1640 medium containing 1 to 20%, preferably 10% to 20% of a fetal bovine serum, a GIT medium (Wako Pure Chemical Industries, Ltd.) containing 1 to 10% of a fetal bovine serum, a serum free medium for cultivation of a hybridoma (SFM-101, Nissui Seiyaku Co., Ltd.), and the like. The culture temperature is normally at from 20 to 40°C, preferably at about 37°C. The culture period is usually for 5 days to 3 weeks, preferably 1 week to 2 weeks. The cultivation can be conducted normally in a 5% carbon dioxide gas. The antibody titer of the culture supernatant of a hybridoma can be determined in a similar way to the determination of the antibody titer in an antiserum as discussed above.

The monoclonal antibody thus obtained can be separated and purified in accordance with publicly known methods, for example, a method of separating and purifying an immunoglobulin [e.g., salting-out, alcohol precipitation, isoelectric point precipitation, electrophoresis, adsorption and desorption with an ion exchanger (e.g., DEAE), ultracentrifugation, gel filtration, or a specific purification method that involves collecting only an antibody with an antigen-binding solid phase or an activated adsorbent such as Protein A, Protein G, or the like, and subsequently dissociating the binding to yield the antibody].

### [Preparation of Polyclonal Antibody]

A polyclonal antibody against the protein used in the present invention can be produced according to publicly known methods, for example, according to a method that involves making an immunogen (antigen of a protein) itself or its complex with a carrier protein; immunizing a warm-blooded animal in a similar way to the above-described method of producing a monoclonal antibody; collecting a product containing an antibody to the protein used in the present invention from the immunized animal; and then separating and purifying the antibody.

With a complex of an immunogen with a carrier protein used to immunize a warm-blooded animal, the kind of the carrier protein and the mixing ratio of a carrier to hapten may arbitrarily be chosen, as long as an antibody is efficiently produced for the hapten immunized by crosslinking to the carrier. For example, a method is used that couples bovine serum albumin, bovine thyroglobulin, hemocyanin, or the like with hapten in a weight ratio of from about 0.1 to about 20:1, preferably from about 1 to 5:1.

In addition, in the coupling of hapten with a carrier, employed are a variety of condensing agents, e.g., glutaraldehyde; carbodiimide; a maleimide activated ester, an activated ester reagent containing a thiol group, a dithiopyridyl group, or the like; etc.

A condensation product is administered to a warm-blooded animal either solely or along with a carrier or a diluent to a site where an antibody can be produce by administration.

In order to enhance antibody productivity when administration, a complete Freund's adjuvant or an incomplete Freund's adjuvant may be administered. The administration is normally made once approximately in 2 to 6 weeks and about 3 to about 10 times in total.

A polyclonal antibody can be collected from blood, ascites, or the like of a warm-blooded animal immunized by the above-described method, preferably from blood of the mammal.

Determination of the polyclonal antibody titer in an antiserum can be made in a similar way to that of the antibody titer in an antiserum as discussed above. Separation and purification of a polyclonal antibody can be carried out in accordance with the method of separating and purifying an immunoglobulin similar to the method of separating and purifying the aforementioned monoclonal antibody.

An antibody to the protein used in the present invention can be inactivated (neutralized) by binding it to the protein or salts thereof used in the present invention, and thus can be utilized as a prophylactic and therapeutic product for diseases associated with the protein used in the present invention as well.

The prophylactic and therapeutic product may be an antibody itself to the protein used in the present invention, but a pharmaceutical composition obtained by admixing the antibody with a pharmaceutically acceptable carrier is preferable. Here, the pharmaceutically acceptable carrier includes a similar carrier to that employed in the above-mentioned "prophylactic and therapeutic product for diseases associated with the protein used in the present invention".

The pharmaceutical composition can be produced in a similar manner to that in the aforementioned "prophylactic and therapeutic product for renal diseases.

A preparation thus obtained is safe and low in toxicity, and thus can be orally or non-orally administered to, for example, mammals (e.g., a human, a mouse, a rat, a rabbit, a sheep, a pig, a bovine, a horse, a cat, a dog, a monkey, a chimpanzee, etc.).

The dose of a prophylactic and therapeutic product for diseases associated with the protein used in the present invention varies depending on a target disease, a target to be administered, a route for administration, or the like. For example, with an adult patient (body weight : 60 kg) suffering from a renal disease, the dose is from about 0.1 to 100 mg, preferably from about 1.0 to about 50 mg, more preferably from about 1.0 to about 20 mg, per day as an effective ingredient, i.e., an antibody to the protein of the present invention.

The protein used in the present invention increases in expression, for example, for renal diseases (e.g., diabetic nephropathy) and the like, and so is useful as a marker in renal diseases for an early diagnosis, criteria for the severity of a symptom, and the prognostication of disease progress. Accordingly, an antibody to the protein used in the present invention can also be employed as a diagnostic product for diseases associated with the protein used in the present invention.

In other words, also disclosed is :
(i) a method for diagnosing a disease associated with the protein or salts thereof used in the present invention, characterized in that the protein or salts thereof used in the present invention in a test solution is quantified by competitively reacting an antibody, the test solution and the labeled protein used in the present invention and by determining the ratio of the labeled protein bonded to the antibody; and
(ii) a method for diagnosing a disease associated with the protein or salts thereof used in the present invention, characterized in that after a test solution, an unsolubilized antibody used in the present invention on a carrier and another labeled antibody used in the present invention are reacted at the same time or continuously, the protein or salts thereof in the test solution is quantified by determining the activity of the labeled agent on the unsolubilized carrier.

In the quantification of (ii) described above, when one antibody is an antibody that recognizes the N terminal region of the protein used in the present invention, the other antibody is desirably an antibody that recognizes the other portion, e.g., the C terminal region, of the protein used in the present invention.

Also, in addition to being capable of quantitatively determining the protein used in the present invention using the monoclonal antibody to the protein, the detection by tissue staining or the like can also be carried out. For these objects, an antibody molecule itself may be used, or a F(ab')₂, Fab' or Fab fraction of an antibody molecule may be utilized as well.

The quantification of the protein or salts thereof used in the present invention using the antibody used in the present invention is not particularly limited. Any determination methods may be used as long as they involve detecting by chemical or physical means the amount of an antibody, an antigen or an antibody-antigen complex corresponding to that of an antigen in a solution measured, and then calculating the amount from a standard curve prepared from standard solutions containing known amounts of antigens. For example, nephrometry, the competitive method, the immunometric method, and the sandwich method are suitably used. The sandwich method discussed below is most preferable from the viewpoint of sensitivity and specificity.

The labeling agent used in determination methods using labeled substances, to be used, include, for example, a radioisotope, an enzyme, a fluorescent substance, a luminescent substance, and the like. The radioisotope for use includes, for example, [¹²⁵I], [¹³¹I], [³H], [¹⁴C], and the like. The aforementioned enzyme for use is preferably stable and high in specific activity, and includes, for example, β-galactosidase, β-glucosidase, an alkaline phosphatase, a peroxidase, malate dehydrogenase, and the like. The fluorescent substance for use includes, for example, fluorescamine, fluorescein isothiocyanate, and the like. The luminescent substance for use includes, for example, luminol, a luminol derivative, luciferin, lucigenin, and the like. Furthermore, the biotin-(strepto) avidin system can be also used for binding an antibody or an antigen to the labeling agent.

In insolubilization of an antigen or antibody, employed is physical adsorption, or a method using chemical bonding normally used for insolubilization or immobilization of a protein. The carrier includes insoluble polysaccharides such as agarose, dextran, cellulose; synthetic resins such as polystyrene, polyacrylamide, silicon; glass; and the like.

The sandwich method involves means that includes reacting an insolubilized monoclonal antibody of the present invention with a test solution (primary reaction), further reacting the resultant material with another labeled monoclonal antibody used in the present invention (secondary reaction), and subsequently determining the activity of the labelling agent on the insolubilized carrier to whereby be capable of quantifying the protein or salts thereof used in the present invention in the test solution. The order of the primary and secondary reactions may be reversed, and the reactions may be carried out simultaneously or with an interval. The labeling agent and the immobilization method can be conducted in accordance with those described above. In addition, in the immunoassay by the sandwich method, an antibody used for an immobilized or labeled antibody is not necessarily one species, but a mixture of two or more species of antibodies may be used in order to improve determination sensitivity.

In the determination method of the protein or salts thereof used in the present invention by the sandwich method, monoclonal antibodies used in the present invention used in the primary and secondary reactions are preferably those whose binding sites by the protein used in the present invention are different. That is, for antibodies used in the primary and secondary reactions, for example, when an antibody used in the secondary reaction recognizes the C terminal region of the protein used in the present invention, an antibody used in the primary reaction preferably recognizes a region exclusive of the C terminal region, e.g. the N terminal region.

A monoclonal antibody used in the present invention can be used for determination systems other than the sandwich method, for example, for the competitive method, the immunometric method, nephrometry, and the like.

The competitive method comprises competitively reacting antigens and labeled antigens in a test solution with antibodies, separating the unreacted labeled antigens (F) from the labeled antigens bound to the antibodies (B) (B/F separation), determining the labeled amount of either of B and F and then quantifying the amount of antigens in the test solution. As this reaction method, employed are a liquid phase method using a soluble antibody as an antibody and using polyethylene glycol and the secondary antibody to the aforementioned antibody or the like for B/F separation, and an immobilized method using an immobilized antibody as the primary antibody, or using a soluble antibody as the primary antibody and using an immobilized antibody as the secondary antibody.

The immunometric method includes competitively reacting antigens in a test solution and immobilized antigens with a specified amount of labeled antibodies, and then separating the immobilized phase from the liquid phase, or includes reacting antigens in a test solution with an excess amount of labeled antibodies, adding immobilized antigens to bind the unreacted labeled antibodies to the immobilized phase, separating the immobilized phase from the liquid phase. Then, the labeled amount in either phase is determined to quantify the amount of antigens in the test solution.

Additionally, the nephrometry determines the amount of insoluble precipitates produced as a result of the antigen-antibody reaction in gel or solution. Even when the amount of antigens in a test solution is small and only a small amount of precipitates is obtained, laser nephrometry using laser scattering is advantageously employed.

Application of these individual immunological determination methods to the quantification methods used in the present invention does not require the setting of any particular conditions, procedures, and the like. A system for determining the protein used in the present invention can be constructed by the addition of the usual technical consideration of a person skilled in the art to the conventional conditions and procedures. For details of these general technical means, review articles, texts, and the like can be referred.

The references include, for example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme Immonoassay" (Igakushoin, published in 1978), Eiji Ishikawa, et al. ed. "Immunoenzyme Assay" (2nd ed.) (Igakushoin, published in 1982), Eiji Ishikawa, et al. ed. "Immunoenzyme Assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies))(all published by Academic Press Publishing).

As discussed so far, use of the antibody used in the present invention enables the protein or salts thereof used in the present invention to be highly sensitively quantified.

In the above-described quantification methods using the antibody used in the present invention, when an excessive increase or decrease of the protein used in the present invention is detected by quantifying the concentration of the protein or the salts thereof in a biopsy sample (e.g., renal cells, or the like) of a test animal, for example, the animal can be diagnosed as being infected by a disease associated with the protein or salts thereof used in the present invention such as a renal disease, or it can be diagnosed as being highly possibly infected in the future.

An antisense nucleotide of DNA encoding the protein used in the present invention, which inhibits translation into the protein by hybridizing a polynucleotide encoding the protein used in the present invention, is useful as a prophylactic and therapeutic product for diseases associated with the protein used in the present invention as in the case of a compound obtained by the screening method of the present invention.

Here, the antisense nucleotide is any one as long as it has complementary or substantially complementary base sequence to a base sequence of DNA encoding the protein used in the present invention, or a partial sequence thereof, and has the expression suppression action on the DNA, with an antisense DNA being preferable.

The base sequence substantially complementary to a base sequence of DNA encoding the protein used in the present invention includes, for example, base sequences that have about 70% or more, preferably about 80% or more, more preferably about 90% or more, most preferably about 95% or more identity to a complete base sequence or a partial base sequence thereof of a base sequence (i.e., a complementary chain of DNA encoding the protein of the present invention) complementary to the base sequence of the DNA, , and the like. In particular, antisense nucleotides are preferable that have about 70% or more, preferably about 80% or more, more preferably about 90% or more, and most preferably about 95% or more identity to a complementary chain of a base sequence (e.g., a base sequence near the initiation codon, or the like) of a portion encoding the N terminal region of the protein used in the present invention among the complete base sequence of the complementary chain of DNA encoding the protein.

Specifically, the antisense nucleotides include antisense nucleotides having complementary or substantially complementary base sequence to a bases sequence of DNA comprising a base sequence represented by SEQ ID NO: 3 or SEQ ID NO: 4, or a portion thereof; preferably, antisense nucleotides having a base sequence complementary to a base sequence comprising a base sequence represented by SEQ ID NO: 3 or SEQ ID NO: 4, or a portion thereof; and the like.

The number of constitution bases of the antisense nucleotide is not particularly limited so long as it inhibits translation into the protein used in the present invention by uniquely hybridizing with a polynucleotide encoding the protein, with the number being normally from about 10 to about 40, preferably from about 15 to about 30.

For the purpose of prevention of decomposition by a hydrolase such as a nuclease, a phosphate of each nucleotide constituting an antisense nucleotide may be replaced by, for example, a chemically modified phosphate such as a phosphonothioate, a methylphosphonate, or phosphonodithioate. In addition, a deoxyribose of each nucleotide may be replaced by a chemically modified deoxyribose of 2'-O-methylation or the like; is subjected to chemical modification in a base portion (pyrimidine or purine) as well; and may also be any deoxyribose as long as it is hybridized with DNA having the base sequence represented by SEQ ID NO: 2. These antisense nucleotides can be produced by means of publicly known DNA synthesis apparatus, or the like.

The antisense nucleotide of DNA encoding the protein used in the present invention is low in toxicity, which can suppress in the body the function (e.g., thyroxine de5'-iodination enzyme activity) of the protein used in the present invention or DNA encoding the protein, can be used as, for example, a prophylactic and therapeutic product for diseases associated with the protein used in the present invention. The antisense nucleotide can be orally or non-orally administered to mammals (e.g., a human, a mouse, a rat, a rabbit, a sheep, a pig, a bovine, a horse, a cat, a dog, a monkey, a chimpanzee, etc.) after processing into a preparation in a similar manner to that in the case of the prophylactic and therapeutic substance for diseases associated with the protein used in the present invention obtained by the screening method using the protein of the present invention. Also, the antisense nucleotide can also be administered after it is inserted into an appropriate vector such as a retrovirus vector, an adenovirus vector, an adenovirus associated virus vector, or the like.

The antisense nucleotide may be administered with a gene gun or a catheter such as a hydrogel catheter, or can also be locally administered into the trachea as an inhalation agent after being made sol.

The dose of the antisense nucleotide varies depending on a target disease, a target to be administered, a route for administration, or the like. For example, with an adult patient (body weight: 60 kg) suffering from a renal disease, the dose is from about 0.1 to about 100 mg, preferably from about 1.0 to about 50 mg, more preferably from about 1.0 to about 20 mg, per day.

Furthermore, an antisense nucleotide of DNA encoding the protein used in the present invention can also be employed as a diagnostic oligonucleotide probe for examining the presence and the expression state of the DNA in a tissue or a cell.

As well as the above-described antisense polynucleotide, a double-stranded RNA comprising a part of RNA encoding the protein used in the present invention, a ribozyme comprising a portion of RNA encoding the protein used in the present invention, or the like can suppress the function of the protein used in the present invention or DNA encoding the protein, and therefore can be used as, e.g., a prophylactic and therapeutic product for renal diseases, or the like.

Here, the double-stranded RNA can be produced by designing on the basis of a sequence of a polynucleotide of the present invention in accordance with publicly known methods, for example, the method described in Nature, 411, 494, 2001.

The ribozyme can be produced by designing on the basis of a sequence of a polynucleotide in accordance with publicly known methods, for example, the method described in TRENDS in Molecular Medicine, 7, 221, 2001. For example, connection of a publicly known ribozyme to a portion of RNA encoding the protein used in the present invention enables production of a desired ribozyme. The portion of RNA encoding the protein used in the present invention includes a portion (RNA fragment) next to a cleaved region of RNA capable of being excised by a publicly known ribozyme.

When the above-mentioned double-stranded RNA or ribozyme is used as a prophylactic and therapeutic product for diseases associated with the protein used in the present invention, it can be formulated as for the above-described antisense polynucleotide for administration.

A polynucleotide which can detect abnormality (gene abnormality) of DNA or mRNA encoding the protein used in the present invention in mammals (e.g., a human, a rat, a mouse, a guinea pig, a rabbit, a sheep, a pig, a bovine, a horse, a cat, a dog, a monkey, a chimpanzee, etc.) by being used as a probe, is useful as a gene diagnosis agent of, e.g., damage to, or mutation or expression decrease of the DNA or mRNA, an increase in or expression excess of the DNA or mRNA, or the like.

The aforementioned gene diagnosis using the polynucleotide can be carried out, for example, by publicly known northern hybridization or the PCR-SSCP method (Genomics), Vol. 5, pp. 874-879 (1989), Proceeding of the National Academy of Sciences of the United States of America, Vol. 86, pp. 2766-2770 (1989), or the like.

For example, when expression excess or decrease is detected by northern hybridization, or if a mutation is detected by the PCR-SSCP method, for example, the subject can be diagnosed as being infected with a disease associated with the protein used in the present invention such as a renal disease, or having a high possibility of being infected with the disease in the future.

Is also disclosed a prophylactic and therapeutic product for diseases associated with the protein used in the present invention, characterized by comprising a polynucleotide having a base sequence represented by SEQ ID NO: 5 or SEQ ID NO: 6. Here, the polynucleotide having a base sequence represented by SEQ ID NO: 5 or SEQ ID NO: 6 is a decoy nucleotide for DNA to which the protein used in the present invention bonds.

The polynucleotide can be produced in accordance with publicly known methods.

The polynucleotide, which is low in toxicity, and which can suppress the function of the protein used in the present invention or DNA encoding the protein (e.g., atranscription controlling activity of a fibrosis relating gene, a cell cycle relating gene and a thrombus relating gene) in the body, can be used as a prophylactic and therapeutic product for diseases associated with the protein used in the present invention. The polynucleotide having a base sequence represented by SEQ ID NO: 5 or SEQ ID NO: 6 can be orally or non-orally administered to mammals (e.g., a human, a mouse, a rat, a rabbit, a sheep, a pig, a bovine, a horse, a cat, a dog, a monkey, a chimpanzee, etc.) after processing into a preparation in a similar manner to that in the case of the prophylactic and therapeutic substance for renal diseases obtained by the screening method using the protein of the present invention.

In addition, the polynucleotide can also be administered after it is inserted into an appropriate vector such as a retrovirus vector, an adenovirus vector, an adenovirus associated virus vector, or the like.

The polynucleotide may be administered with a gene gun or a catheter such as a hydrogel catheter, or can also be locally administered into the trachea as an inhalation agent after being made sol.

The dose of the prophylactic and therapeutic product for diseases associated with the protein used in the present invention varies depending on a target disease, a target to be administered, a route for administration, or the like. For example, with an adult patient (body weight : 60 kg) suffering from a renal disease, the dose is from about 0.1 to about 100 mg, preferably from about 1.0 to about 50 mg, more preferably from about 1.0 to about 20 mg, per day as an effective ingredient, i.e., a nucleotide having a base sequence represented by SEQ ID NO: 5 or SEQ ID NO: 6.

Is also disclosed a prophylactic and therapeutic product for diabetic nephropathy comprising a suppressor of Egr-1.

The suppressor of Egr-1 is not particularly limited as long as it is a substance capable of suppressing production or expression of Egr-1, or the activity of Egr-1 in the body, and may be any of a peptide, a protein, a non-peptide compound, a synthetic compound, a fermented product, a cell extract, a plant extract, an animal tissue extract, plasma, or the like. These may form salts, and examples of the salt include salts similar to those of the protein of the present invention described above.

The suppressor of Egr-1 is preferably a substance capable of suppressing production or expression of Egr-1, or the activity of Egr-1 in kidneys, and thus a suppressor of renal Egr-1.

The suppressor of Egr-1 may also be a substance capable of suppressing production or expression of factors such as a fibrosis relating gene, a cell cycle relating gene, and a thrombus relating gene in the body, or suppressing the activities of the factors.

Here, the fibrosis relating gene, the cell cycle relating gene, and the thrombus relating gene include those illustrated as the above-mentioned "substantially the same activity."

A prophylactic and therapeutic product for diabetic nephropathy can be formulated as for the prophylactic and therapeutic substance for renal diseases.

The prophylactic and therapeutic product for diabetic nephropathy is safe and low in toxicity, and thus can be orally or non-orally administered to, for example, mammals (e.g., a human, a mouse, a rat, a rabbit, a sheep, a pig, a bovine, a horse, a cat, a dog, a monkey, a chimpanzee, etc.).

The dose of the prophylactic and therapeutic product for diabetic nephropathy varies depending on a target disease, a target to be administered, a route for administration, or the like. For example, with an adult patient (body weight: 60 kg), the dose is from about 0.1 to about 100 mg, preferably from about 1.0 to about 50 mg, more preferably from about 1.0 to about 20 mg, per day as an effective ingredient, i.e., a suppressor of Egr-1.

Herein, representation of a base, an amino acid, or the like by a code is in accordance with a code based on IUPAC-IUB Commission on Biochemical Nomenclature or a conventional code in the art, with examples thereof being indicated below. Also, when an optical isomer on an amino acid is present, it indicates the L form unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP: : deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: : leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- PGlu: : pyroglutamic acid
- Sec: : selenocysteine

The sequence identification numbers in the sequence listing of the description indicates the sequences below.
[SEQ ID NO: 1]
   This represents a partial sequence of amino acid sequences of Egr-1 preserved in a human, a mouse and a rat.
[SEQ ID NO: 2]
   This represents an amino acid sequence of human Egr-1.
[SEQ ID NO: 3]
   This represents a base sequence of DNA encoding human Egr-1 having an amino acid sequence represented by SEQ ID NO: 2.
[SEQ ID NO: 4]
   This represents a base sequence of DNA encoding human Egr-1 having an amino acid sequence represented by SEQ ID NO: 2.
[SEQ ID NO: 5]
   This represents a base sequence of a decoy nucleotide of Egr-1.
[SEQ ID NO: 6]
   This represents a base sequence of a decoy nucleotide of Egr-1.
[SEQ ID NO: 7]
   This represents a base sequence of Egr-1 antisense oligonucleotide.
[SEQ ID NO: 8]
   This represents a base sequence of a primer to amplify Egr-1 cDNA.
[SEQ ID NO: 9]
   This represents a base sequence of a primer to amplify Egr-1 cDNA.
[SEQ ID NO: 10]
   This represents a base sequence of a sense strand of double-stranded DNA capable of binding to Egr-1.
[SEQ ID NO: 11]
   This represents a base sequence of a sense strand of double-stranded DNA not capable of binding to Egr-1.

Hereinafter, the present invention will in more detail be set forth in terms of the EXAMPLES and EXPERIMENTAL EXAMPLES. However, the present invention should not be limited thereto.

### EXAMPLES

### EXPERIMENTAL EXAMPLE 1

### Increase in the early growth response-1 (Egr-1) mRNA expression in the kidney of Wistar fatty rats

Using 13, 22 and 40-week-old male Wistar fatty rats (Takeda Rabics) that show non-insulin dependent diabetes mellitus (NIDDM) and spontaneously develop diabetic nephropathy and their normal control male Wistar lean rats (Takeda Rabics) that have the same week-age, urine was accumulated for 24 hours and blood was collected from tail vein to determine the urinary albumin excretion, the concentrations of glucose and insulin in the plasma. The urinary albumin excretion was determined with A/G B Test Wako (Wako Pure Chemical Industries); the concentrations of glucose in the plasma were determined with Sychron CX5 Delta (Beckman Coulter). The concentrations of insulin were determined by the RIA method (Shionogi & Co. Ltd.).

The kidneys were collected from five rats of each case and stored at -80°C, and then were crushed to extract total RNA. The primers and fluorescent probe were made on the basis of the mRNA sequence previously reported, and subsequently the amount of each of individual mRNAs was determined using ABI PRISM 7700 (Applied Biosystems) by the real time quantitative RT-PCR method.

The results are shown in [Table 1] and [Table 2]. The Wistar fatty rat showed NIDDM from the age of 13 weeks and developed DN from the age of 22 weeks; as a result, the urinary albumin excretion was increased. It showed an increase in the level of Egr-1 mRNA expression after the age of 22 weeks, and increases in the level of expression of Transforming growth factor-β1 (TGF-β1) and fibronectin mRNA at the age of 40 weeks.

**[Table 1] Urinary albumin excretion, concentration of glucose in plasma and concentration of insulin in plasma**

| | Wistar lean rat | | | Wistar fatty rat | | |
|---|---|---|---|---|---|---|
| weeks age | 13 | 22 | 40 | 13 | 22 | 40 |
| Urinary albumin excretion (mg/day) | 5.1 | 4.0 | 10.4 | 6.7 | 55.7 | 182.0 |
| Concentration of glucose in plasma (mg/dl) | 138.9 | 137.1 | 134.3 | 319.2 | 402.9 | 319.4 |
| Concentration of insulin in plasma (µUnits/ml) | 123.0 | 125.6 | 158.7 | 1091.9 | 1019.2 | 1674.4 |

| | | | | | | |
|---|---|---|---|---|---|---|
| (n=5, Mean) | | | | | | |

**[Table 2] The mRNA expression levels of Egr-1, TGF-β1 and fibronectin in the kidney of Wistar fatty rats (Relative values when the expression level in the kidney of Wistar lean rats is equal to one)**

| Weeks age | 13 | 22 | 40 |
|---|---|---|---|
| Egr-1 | 1.1 | 2.2 | 2.4 |
| TGF-β1 | 1.1 | 1.0 | 1.7 |
| Fibronectin | 1.2 | 1.0 | 2.1 |

| | | | |
|---|---|---|---|
| (n=5, Mean) | | | |

### EXPERIMENTAL EXAMPLE 2

### Changes in the early growth response-1 (Egr-1) mRNA expression in the kidney of Zucker fatty rats

To a male Zucker fatty rat (ZF rat, 18 weeks age, Charles River Japan Inc.) that shows hyperinsulinemia and spontaneously develops a renal damage, Candesartan cilexetil (angiotensin II type 1 receptor antagonist) suspended in 0.5% methylcellulose 100cP was orally administered once per day everyday for 9 weeks. To the control group and the normal control group, i.e., male Zucker lean rats (ZL rat, Charles River Japan Inc.) with the same age, 0.5% methylcellulose 100cP (Vehicle) was administered once per day everyday. Eight weeks after administration, urine was accumulated for 24 hours, and then the urinary albumin excretion was determined using A/G B Test Wako (Wako Pure Chemical Industries). Nine weeks after administration, the kidneys were collected and stored at -80°C, and subsequently the kidneys were crushed to extract the total RNA. The primers and fluorescent probe were prepared on the basis of the mRNA sequence previously reported, and then the amount of each of individual mRNAs was determined using ABI PRISM 7700 (Applied Biosystems) by the real time quantitative RT-PCR method.

The results are shown in [Table 3] and [Table 4]. In the kidney from the Zucker fatty rat in which the urinary albumin excretion is increased, the amount of Egr-1 mRNA expression is increased and also each mRNA expression of Transforming growth factor-β1 (TGF-β1), platelet-derived growth factor-B (PDGF-B), fibronectin and α1(I) collagen is increased. Furthermore, inhibition of an increase in the urinary albumin excretion by Candesartan cilexetil administration also inhibits the increase in the level of any of the aforementioned mRNA expression.

**[Table 3] Urinary albumin excretion, concentration of glucose in blood and concentration of insulin in blood**

| | ZL rat Vehicle | ZF rat Vehicle | ZF rat Candesartan cilexetil |
|---|---|---|---|
| Urinary albumin excretion (mg/day) | 48.5 | 401.5 | 61.7 |

| | | | |
|---|---|---|---|
| (n=8 - 9, Mean) | | | |

**[Table 4] The mRNA expression levels of Egr-1, TGF-β1, PDGF-B, fibronectin and α1 (I) collagen in the kidney of Zucker Fatty Rats (Relative values when the expression level in the kidney of the ZL rat is equal to one)**

| | ZF rat Vehicle | ZF rat Candesartan |
|---|---|---|
| Egr-1 | 2.9 | 1.3 |
| TGF-β1 | 2.1 | 1.2 |
| PDGF-B | 1.5 | 1.0 |
| Fibronectin | 2.1 | 1.1 |
| α1 (I) collagen | 2.3 | 1.6 |

| | | |
|---|---|---|
| (n=8 - 9, Mean) | | |

### EXPERIMENTAL EXAMPLE 3

### Increase in the early growth response-1 (Egr-1) mRNA expression in rat glomerular mesangial cells

Glomerular mesangial cells (passege 7) isolated and cultivated from a male Sprague-Dawley rat (SD rat, Japan Crea) of the age of 4 weeks were cultivated in DMEM medium containing 20% fetal calf serum (FCS) for 4 days, and then the cells were cultivated in DMEM containing 0.2% bovine serum albumin for 3 days.

To the cells after cultivation, FCS (20%) or angiotensin II (AII) (10⁻⁶M) was added, and then the resulting mixture was reacted at 37°C for 30 minutes. The AII type 1 receptor antagonist Candesartan of 10⁻⁵ M as the final concentration was added to the medium 5 minutes prior to the stimulation of FCS or AII. Thirty minutes after FCS or AII stimulus, total RNA was extracted. The primers and fluorescent probe were prepared on the basis of the mRNA sequence previously reported, and subsequently the level of Egr-1 mRNA expression was determined using ABI PRISM 7700 (Applied Biosystems) by the real time quantitative RT-PCR method.

The results are shown in [Table 5] and [Table 6]. The amount of Egr-1 mRNA expression was remarkably increased 30 minutes after FCS stimulus, and was partially inhibited with candesartan. Also, 30 minutes after AII stimulus, the amount of Egr-1 mRNA expression was slightly increased, and was mostly suppressed with Candesartan.

**[Table 5] Increase in the Egr-1 mRNA expression by FCS Stimulus and inhibitory effect by Candesartan (Relative values when the expression level before stimulus is equal to one)**

| | Egr-1 mRNA expression level |
|---|---|
| Before stimulus | 1.0 |
| 30 minutes after FCS stimulus + Vehicle | 26.6 |
| 30 minutes after FCS stimulus + Candesartan | 18.4 |

| | |
|---|---|
| (n=3, Mean) | |

**[Table 6] Increase in the Egr-1 mRNA expression by AII Stimulus and inhibitory effect by Candesartan (Relative values when the expression level before stimulus is equal to one)**

| | Egr-1 mRNA expression level |
|---|---|
| Before stimulus | 1.0 |
| 30 minutes after AII stimulus + Vehicle | 2.8 |
| 30 minutes after AII stimulus + Candesartan | 1.3 |

| | |
|---|---|
| (n=3, Mean) | |

### EXPERIMENTAL EXAMPLE 4

### Increase in the early growth response-1 (Egr-1) mRNA in the kidney of spontaneously hypercholesterolemic rats

Using male spontaneously hypercholesterolemic rats (SHC rats, 11 weeks age, Takeda Rabies), which develop renal damage spontaneously, and normal control male Sprague-Dawley rats (SD rat, Crea Japan Inc.) that have the same week-age, urine was accumulated for 24 hours and blood was collected from tail vein to determine the urinary albumin excretion and the total cholesterol in the plasma. The urinary albumin excretion was determined with A/G B Test Wako (Wako Pure Chemical Industries, Ltd.); Total cholesterol concentrations in the plasma were determined using Sychron CX5 Delta (Beckman Coulter).

The kidneys were collected at 12 weeks age and stored at -80°C, and then were crushed to extract total RNA. The primers and fluorescent probe were made on the basis of the mRNA sequence previously reported, and subsequently the levels of Egr-1 mRNA expression were determined using ABI PRISM 7700 (Applied Biosystems) by the real time quantitative RT-PCR method.

The urinary albumin excretion for 12-week-old SHC rats and SD rats were, respectively, 179.2 and 9.2 (mg/day, Mean (n = 5)), and the concentrations of cholesterol in the plasma were, respectively, 92.4 and 43.5 (mg/dl, Mean (n = 5)), with any of the parameters in the SHC rats being high. The amounts of Egr-1 mRNA expression in the kidneys of the SHC rats were increased 3.8 times (Mean (n = 5)) those of Egr-1 mRNA expression in the kidneys of the SD rats.

### EXPERIMENTAL EXAMPLE 5

### Induction for expression of renal fibrosis-related genes by the early growth response (Egr)-1 overexpression in HEK-293 cells derived from human fetal kidney

HEK-293 cells (CRL-1573, ATCC, passege 40) derived from a human fetal kidney was cultivated for a day in DMEM medium containing 10% fetal bovine serum, and then a human Egr-1 expression plasmid was transfected into the cell at the concentration of 2 µg/dish using 20 µg/3.5 cm dish of Polyfect (QIAGEN). In addition, as the Egr-1 expression plasmid, a plasmid in which cDNA of the coding region of human Erg-1 was inserted into the pBK-CMV vector (STRATAGENE) was used. Only the pBK-CMV vector used in the Egr-1 plasmid preparation was incorporated into the control cell. 24 Hours after transfection, the cells were harvested to extract protein and total RNA. For the Egr-1 protein expression, after conduct of SDS-PAGE, Western blotting using the Egr-1 specific antibody (Anti-Egr-1 (C-19), SantaCruz) was performed, and then the bands were detected by the chemoluminescence technique (PIERCE). The brightness accumulation value of the band thus detected was calculated by a CS Analyzer 1.00a (ATTO). Also, the levels of mRNA expression of the renal fibrosis-related genes were determined by preparing the primer and fluorescent probe on the basis of the mRNA sequence previously reported and by using ABI PRISM 7700 (Applied Biosystems) by means of the real time quantitative RT-PCR method.

The results are shown in [Table 7]. The transfection of the Egr-1 expression plasmid increases the Egr-1 protein expression and also the mRNA expression levels of the renal fibrosis-related genes such as tissue factor, fibronectin, and intercellular adhesion molecule (ICAM)-1.

**[Table 7] Increase in the Egr-1 protein expression and increase in the expression of renal fibrosis-related genes by transfection of Egr-1 plasmid (Relative values when the expression level of control is equal to one)**

| | Human Egr-1 expression plasmid-transfected cells |
|---|---|
| Egr-1 protein | 1.5 |
| Tissue factor mRNA | 1.6 |
| Fibronectin mRNA | 1.3 |
| ICAM-1 mRNA | 4.9 |

| | |
|---|---|
| (n=3 - 6, Mean) | |

### EXAMPLE 1

### Study on the effect of the early growth response-1 (Egr-1) antisense oligodeoxynucleotide (AS-ODN) in rat glomerular mesangial cells

Glomerular mesangial cells (passege 4-5) isolated and cultivated from a male SD rat with 4 weeks age were cultivated for 3 to 4 days in DMEM medium containing 20% fetal bovine serum to sub-confluence, and the medium was changed to DMEM containing a 0.2% bovine serum albumin and then the resulting material was subjected to serum starvation. Forty-eight hours after initiation of the serum starvation, Egr-1 AS-ODN (SEQ ID NO: 7) or a Control-ODN (the scrambled ODNs that were matched with the AS-ODN in size and the number of bases, both synthesized by BIOGNOSTIK Corp.) with a concentration of 20 µM was added to the medium and the resultant material was allowed to stand in a CO₂ incubator for 24 hours. Thereafter the material was stimulated with a basic fibroblast growth factor (bFGF) (3 ng/ml), and then was reacted at 37°C for 1 to 2 hours. A protein was extracted from the cell one hour after bFGF stimulus, and total RNA was extracted from a cell two hours after bFGF stimulus. The expression of the Egr-1 protein, after conduct of SDS-PAGE, was subjected to Western blotting using the Egr-1 specific antibody (Anti-Egr-1 (C-19), SantaCruz) to detect bands by the chemoluminescence technique (PIERCE). The brightness accumulation value of the band thus detected was calculated by a CS Analyzer 1.00a (ATTO). Also, the mRNA expression level of the tissue factor was determined by making the primer and fluorescent probe on the basis of the mRNA sequence previously reported and by using ABI PRISM 7700 (Applied Biosystems) by means of the quantitative RT-PCR method.

The results are shown in [Table 8]. The Egr-1 protein expression one hour after bFGF stimulus was increased up to 5.1-fold and this increase was suppressed by Egr-1 AS-ODN treatment. Inhibitory action was not observed at all by Control-ODN treatment. In addition, the tissue factor mRNA was increased up to 2.5-fold, and this increase was almost completely suppressed by Egr-1 AS-ODN treatment. Inhibitory action was not recognized in the Control-ODN treatment group. Furthermore, cytotoxicity by transfection of the Egr-1 AS-ODN was not observed.

**[Table 8] The effects of the Egr-1 AS-ODN transfection on the expression of the Egr-1 protein and the expression of tissue factor mRNA after bFGF stimulus(Relative values when the expression level of control is equal to one)**

| Stimulus | Control | bFGF (3 ng/ml) | | |
|---|---|---|---|---|
| Treatment | Vehicle | Vehicle | AS-ODN | Control-ODN |
| Egr-1 protein | 1.0 | 5.1 | 2.0 | 7.0 |
| Tissue factor mRNA | 1.0 | 2.5 | 1.1 | 4.6 |

| | | | | |
|---|---|---|---|---|
| (n=2 - 3, Mean) | | | | |

### EXAMPLE 2

### Quantification of the Egr-1 protein utilizing DNA binding activity

A system of quantifying the Egr-1 protein with a microplate was set by the combination of synthetic DNA having the Egr-1 binding sequence and the anti-Egr-1 antibody.

### (1) Construction of plasmids for Egr-1 expression and expression thereof in animal cells

In order to obtain the human Egr-1 cDNA, a polymerase chain reaction was carried out using as a template cDNA (Clontech Corp.) derived from human fat tissues and synthetic DNA (SEQ ID NOS: 8 and 9) as a primer. The terminal portion of the amplified cDNA was cleaved by the restriction enzymes EcoRI and PstI, and the fragment thus obtained was cloned into the plasmid pcDNA3.1 (+) (Invitrogen Corp.) cleaved by EcoRI and PstI to confirm the base sequence.

In petri dishes for cultivation (3003, FALCON Corp.) were each planted the cultivated cell HEK293 derived from human with a number of 2 x 10⁶ using as a medium DMEM containing 10% FCS and 100 µg/ml of kanamycin. On the next day, the gene was introduced into cells using 20 µg of the aforementioned plasmid for Egr-1 expression and Lipofectamine 2000 (Invitrogen Corp.). After 48 hours, the medium was removed, and the resulting cells were washed with PBS, and then to these were added a 0.5 ml of lysis solution (solution produced by adding to an aqueous solution containing 25 mM Tris-HCl (pH 7.4), 0.4 M NaCl, 100 µM EDTA, 1 mM DTT and 1% TritonX-100, 1/100 volume of Protease inhibitor cocktail (SIGMA Corp. P8340)) to lyse the cells, which were collected in a sample tube. After the tube was allowed to stand on ice for 5 minutes, the cells were agitated for 10 seconds with a Voltex mixer and centrifuged at 12000xg at 4°C for 5 minutes to obtain the supernatant. This was used as the standard sample. Similarly, the vector, pcDNA3.1(+) was also introduced. The cell lysate was used as the control.

### (2) Detection of Egr-1

The Egr-1 binding sequence-containing synthetic DNA (SEQ ID NO: 10) in which the 5' terminus was labeled with biotin, was made to form the double-strands with the synthetic DNA having the complementary strand (the complementary strand was not labeled with biotin). Also, the synthetic DNA (SEQ ID NO: 11) into which a base substituent hindering the binding of Egr-1 was introduced was also made to form the double-stranded chain, which was used as the control. These were diluted to a final concentration of 50 nM with Block Ace (containing 1mM EDTA) (product of Snow Brand Milk Products Co., Ltd., purchased from Dainippon Pharmaceutical Co. Ltd.) diluted 4-fold with PBS, and 100 µl aliquots each was added to 96-well plates (IWAKI) immobilized with streptoadipin. After the resulting plate was allowed to stand at room temperature for 30 minutes, the solution was removed by suction, to the resultant plate was added 300 µl of Block Ace (containing 1mM EDTA) diluted 4-fold with PBS and the material thus obtained was allowed to stand at room temperature for 3 hours. After removal of the solution, the resultant plate was washed 3 times with 300 µl of PBS. To each well of the plate thus obtained was added 100 µl of a standard sample diluted 50-fold with a measuring buffer [50 mM Tris-HCl (pH 7.4), 100 mM NaCl, 10% Block Ace, 200 µM ZnSO₄, 250 µM MDTT, 10 µg/ml poly dI-dC (Amersham Corp.), 0.05% Triton X-100] or 100 µl of the control sample (prepared in (1) described above). After the resultant plate was allowed to stand at room temperature for 40 minutes, it was washed 3 times with a 300 µl of a washing solution (2 mM imidazole-buffered saline, 0.02% Tween 20, 200 µM ZnSO₄). To each well of the plate thus obtained was added 100 µl of anti-Egr-1 antigen (rabbit, polyclonal antibody, SANTA CRUZ Corp., sc-189) that was diluted 500-fold with a measuring buffer not containing poly dl-dC, and the resulting plate was allowed to stand at 4°C for 5 hours. The plate thus obtained was washed 3 times with the 300 µl washing solution, and to each well of the plate was added 100 µl of anti rabbit IgG antigen (HRP labeling, Cell Signaling Corp.) that was diluted 2000-fold with a measuring buffer not containing poly dl-dC, and thereafter the resultant plate was allowed to stand at 4°C overnight. The plate thus obtained was washed 6 times with the 300 µl washing water, followed by staining the well material by adding 100 µl of a TMB solution (DAKO Corp.) and stopping the reactions by adding 100 µl of 2N sulfuric acid. The products in the well were determined for absorbance at 450 nm on an photometer (TECAN Corp., Spectrarainbow).

The absorbance when a dissolution solution of cells into which Egr-1 cDNA was introduced was used by means of a plate on which synthetic DNA of a sequence capable of binding Egr-1 was 0.751. The absorbance when the same sample was determined using a plate on which DNA of non-binding sequence was immobilized was 0.016, and the absorbance using a plate without immobilizing DNA was 0.005. The absorbance value when a measurement was carried out using cell lysate into which Egr-1 cDNA was not introduced, with a plate on which DNA of binding sequence was immobilized, was 0.005.

### INDUSTRIAL APPLICABILITY

The screening method of the present invention enables screening of a prophylactic and therapeutic product for renal diseases with excellent effects and no adverse reactions.

## Claims

1. A screening method for a prophylactic and therapeutic substance for a renal disease, which is **characterized by**:
cultivating a cell that has the capability of producing a protein or salts thereof comprising an amino acid sequence represented by SEQ ID NO: 2 in the absence and the presence of a test compound,
immobilizing on a solid phase a polynucleotide, to which the protein or salts thereof is capable of binding,
adding to the solid phase the protein or salts thereof and an antibody against the protein or salts thereof,
measuring and comparing the production of the protein or salts thereof, and a binding activity of the protein or salts thereof to the polynucleotide in the case where the cell is cultivated in the absence of the test compound and the case where the cell is cultivated in the presence of the test compound; and
selecting a compound that decreases the amount of the protein or salts thereof and/or a compound that decreases the binding activity of the protein or salts thereof to the polynucleotide.

2. The screening method according to claim 1, wherein the renal disease is an Egr-1 depending renal disease.

3. The screening method according to claim 1, wherein the renal disease is diabetic nephropathy.

## Patentansprüche

1. Screening-Verfahren für eine prophylaktische und therapeutische Substanz für eine Nieren-Krankheit, das **gekennzeichnet ist durch**:
Kultivieren einer Zelle, die in der Lage ist, ein Protein oder Salze davon, umfassend eine Aminosäure-Sequenz, wiedergegeben **durch** SEQ ID NR. 2, in Abwesenheit oder in Gegenwart von einer Testverbindung zu erzeugen,
Immobilisieren an einer festen Phase eines Polynukleotids, an das das Protein oder die Salze davon blinden können,
Zusetzen des Proteins oder der Salze davon und eines Antikörpers gegen das Protein oder die Salze davon zu der festen Phase;
Messen und Vergleichen der Erzeugung des Proteins oder der Salze davon und einer Bindungsaktivität des Proteins oder der Salze davon an das Polynukleotid in dem Fall, wenn die Zelle in Abwesenheit der Testverbindung kultiviert wird, und dem Fall, wenn die Zelle in Gegenwart der Testverbindung kultiviert wird; und
Auswählen einer Verbindung, die die Menge von dem Protein oder den Salzen davon senkt und/oder einer Verbindung, die die Bindungsaktivität des Proteins oder der Salze davon an das Polynukleotid senkt.

2. Screening-Verfahren nach Anspruch 1, wobei die Nieren-Krankheit eine Egr-1 abhängige Nieren-Krankheit ist.

3. Screening-Verfahren nach Anspruch 1, wobei die Nieren-Krankheit diabetische Nephropathie ist.

## Revendications

1. Procédé de recherche par criblage d'une substance thérapeutique et prophylactique pour une maladie rénale, **caractérisé par** les étapes suivantes:
- cultiver, en présence ou en l'absence d'un composé testé, une cellule qui est capable de produire une protéine comprenant la séquence d'acides aminés présentée en tant que Séquence n° 2, ou un sel d'une telle protéine ;
- immobiliser sur une phase solide un polynucléotide auquel ladite protéine ou ses sels sont capables de se lier ;
- ajouter à cette phase solide de la protéine ou de l'un de ses sels, ainsi qu'un anticorps dirigé contre la protéine ou ses sels ;
- mesurer la production de la protéine ou de son sel et l'activité de liaison de la protéine ou de son sel au polynucléotide, dans le cas où la cellule est cultivée en l'absence du composé testé et dans le cas où la cellule est cultivée en présence du composé testé, et comparer les résultats obtenus dans ces deux cas ;
- et sélectionner un composé qui fait diminuer la quantité de protéine ou de son sel produite et/ou qui fait diminuer l'activité de liaison de la protéine ou de son sel au polynucléotide.

2. Procédé de recherche par criblage conforme à la revendication 1, ladite maladie rénale étant une maladie rénale dépendante du facteur Egr-1.

3. Procédé de recherche par criblage conforma à la revendication 1, ladite maladie rénale étant une néphropathie diabétique.
